# EUROPEAN PATENT SPECIFICATION

(11) **EP 2 493 288 B1**
(45) Date of publication and mention of the grant of the patent: **18.02.2015**
(21) Application number: 10771121.0
(22) Date of filing: 28.10.2010
(51) Int. Cl.: A01K 67/027, C12N 9/22, C12N 15/85, C12N 15/90

(54) **HOMOLOGOUS RECOMBINATION IN THE OOCYTE**
HOMOLOGE REKOMBINATION IN DER EIZELLE
RECOMBINAISON HOMOLOGUE DANS L'OOCYTE

(30) Priority: 28.10.2009 US 255621 P
(43) Date of publication of application: 05.09.2012
(73) Proprietor: Helmholtz Zentrum München Deutsches Forschungszentrum für Gesundheit und Umwelt (GmbH), 85764 Neuherberg (DE)
(72) Inventor: KÜHN, Ralf, 13158 Berlin (DE); WURST, Wolfgang, 80937 München (DE); MEYER, Melanie, 85375 Neufahrn (DE)
(74) Representative: Vossius & Partner
(86) International application number: PCT/EP2010/066357
(87) International publication number: WO 2011/051390

(56) References cited:
- WO-A2-03/087341
- BOZAS ANA ET AL: "Genetic Analysis of Zinc-Finger Nuclease-Induced Gene Targeting in Drosophila", GENETICS, vol. 182, no. 3, July 2009 (2009-07), pages 641-651, XP009143061, ISSN: 0016-6731
- BIBIKOVA M ET AL: "Stimulation of homologous recombination through targeted cleavage by chimeric nucleases", MOLECULAR AND CELLULAR BIOLOGY, AMERICAN SOCIETY FOR MICROBIOLOGY, WASHINGTON, US, vol. 21, no. 1, 1 January 2001 (2001-01-01), pages 289-297, XP002974229, ISSN: 0270-7306, DOI: DOI:10.1128/MCB.21.1.289-297.2001
- PORTEUS MATTHEW: "Design and testing of zinc finger nucleases for use in mammalian cells", METHODS IN MOLECULAR BIOLOGY HUMANA PRESS INC, 999 RIVERVIEW DR, STE 208, TOTOWA, NJ 07512-1165 USA SERIES : METHODS IN MOLECULAR BIOLOGY (ISSN 1064-3745(PRINT)), 2008, pages 47-61, XP9143028,
- ZOU JIZHONG ET AL: "Gene Targeting of a Disease-Related Gene in Human Induced Pluripotent Stem and Embryonic Stem Cells", CELL STEM CELL, vol. 5, no. 1, July 2009 (2009-07), pages 97-110, XP009143029, ISSN: 1934-5909
- HOCKEMEYER DIRK ET AL: "Efficient targeting of expressed and silent genes in human ESCs and iPSCs using zinc-finger nucleases.", NATURE BIOTECHNOLOGY SEP 2009 LNKD- PUBMED:19680244, vol. 27, no. 9, September 2009 (2009-09), pages 851-857, XP009143038, ISSN: 1546-1696
- SANTIAGO YOLANDA ET AL: "Targeted gene knockout in mammalian cells by using engineered zinc-finger nucleases.", PROCEEDINGS OF THE NATIONAL ACADEMY OF SCIENCES OF THE UNITED STATES OF AMERICA 15 APR 2008 LNKD- PUBMED:18359850, vol. 105, no. 15, 15 April 2008 (2008-04-15), pages 5809-5814, XP009143037, ISSN: 1091-6490
- PORTEUS M H ET AL: "GENE TARGETING USING ZINC FINGER NUCLEASES", NATURE BIOTECHNOLOGY, NATURE PUBLISHING GROUP, NEW YORK, NY, US, vol. 23, no. 8, 1 August 2005 (2005-08-01) , pages 967-973, XP002467422, ISSN: 1087-0156, DOI: DOI:10.1038/NBT1125
- MEYER MELANIE ET AL: "Gene targeting by homologous recombination in mouse zygotes mediated by zinc-finger nucleases.", PROCEEDINGS OF THE NATIONAL ACADEMY OF SCIENCES OF THE UNITED STATES OF AMERICA 24 AUG 2010 LNKD- PUBMED:20686113, vol. 107, no. 34, 24 August 2010 (2010-08-24), pages 15022-15026, XP009143027, ISSN: 1091-6490

### SEQUENCE LISTING

<110> Helmholtz Zentrum München
<120> Homologous recombination in the oocyte
<130> R2816 PCT
<150> US 61/255,621
   <151> 2009-10-28
<160> 15
<170> PatentIn version 3.4
<210> 1
   <211> 12565
   <212> DNA
   <213> artificial sequence
<220>
   <223> /note="Description of artificial sequence: gene targeting vector pRosa26.8-2"
<400> 1
   caccgcatta ccctgttatc cctagcggca ggccctccga gcgtggtgga gccgttctgt      60
   gagacagccg ggtacgagtc gtgacgctgg aaggggcaag cgggtggtgg gcaggaatgc     120
   ggtccgccct gcagcaaccg gagggggagg gagaagggag cggaaaagtc tccaccggac     180
   gcggccatgg ctcggggggg ggggggcagc ggaggagcgc ttccggccga cgtctcgtcg     240
   ctgattggct tcttttcctc ccgccgtgtg tgaaaacaca aatggcgtgt tttggttggc     300
   gtaaggcgcc tgtcagttaa cggcagccgg agtgcgcagc cgccggcagc ctcgctctgc     360
   ccactgggtg gggcgggagg taggtggggt gaggcgagct ggacgtgcgg gcgcggtcgg     420
   cctctggcgg ggcgggggag gggagggagg gtcagcgaaa gtagctcgcg cgcgagcggc     480
   cgcccaccct ccccttcctc tgggggagtc gttttacccg ccgccggccg ggcctcgtcg     540
   tctgattggc tctcggggcc cagaaaactg gcccttgcca ttggctcgtg ttcgtgcaag     600
   ttgagtccat ccgccggcca gcgggggcgg cgaggaggcg ctcccaggtt ccggccctcc     660
   cctcggcccc gcgccgcaga gtctggccgc gcgcccctgc gcaacgtggc aggaagcgcg     720
   cgctgggggc ggggacgggc agtagggctg agcggctgcg gggcgggtgc aagcacgttt      780
   ccgacttgag ttgcctcaag aggggcgtgc tgagccagac ctccatcgcg cactccgggg     840
   agtggaggga aggagcgagg gctcagttgg gctgttttgg aggcaggaag cacttgctct     900
   cccaaagtcg ctctgagttg ttatcagtaa gggagctgca gtggagtagg cggggagaag     960
   gccgcaccct tctccggagg ggggagggga gtgttgcaat acctttctgg gagttctctg    1020
   ctgcctcctg gcttctgagg accgccctgg gcctgggaga atcccttccc cctcttccct    1080
   cgtgatctgc aactccagtc tttctaggcg cgccctcgag gtgacctgca cgtctagggc 1140
   gcagtagtcc agggtttcct tgatgatgtc atacttatcc tgtccctttt ttttccacag 1200
   ctcgcggttg aggacaaact cttcgcggtc tttccagtac taggggatcg aaagagcctg 1260
   ctaaagcaaa aaagaagtca ccatgtcgtt tactttgacc aacaagaacg tgattttcgt 1320
   tgccggtctg ggaggcattg gtctggacac cagcaaggag ctgctcaagc gcgatcccgt 1380
   cgttttacaa cgtcgtgact gggaaaaccc tggcgttacc caacttaatc gccttgcagc 1440
   acatccccct ttcgccagct ggcgtaatag cgaagaggcc cgcaccgatc gcccttccca 1500
   acagttgcgc agcctgaatg gcgaatggcg ctttgcctgg tttccggcac cagaagcggt 1560
   gccggaaagc tggctggagt gcgatcttcc tgaggccgat actgtcgtcg tcccctcaaa 1620
   ctggcagatg cacggttacg atgcgcccat ctacaccaac gtgacctatc ccattacggt 1680
   caatccgccg tttgttccca cggagaatcc gacgggttgt tactcgctca catttaatgt 1740
   tgatgaaagc tggctacagg aaggccagac gcgaattatt tttgatggcg ttaactcggc 1800
   gtttcatctg tggtgcaacg ggcgctgggt cggttacggc caggacagtc gtttgccgtc 1860
   tgaatttgac ctgagcgcat ttttacgcgc cggagaaaac cgcctcgcgg tgatggtgct 1920
   gcgctggagt gacggcagtt atctggaaga tcaggatatg tggcggatga gcggcatttt 1980
   ccgtgacgtc tcgttgctgc ataaaccgac tacacaaatc agcgatttcc atgttgccac 2040
   tcgctttaat gatgatttca gccgcgctgt actggaggct gaagttcaga tgtgcggcga 2100
   gttgcgtgac tacctacggg taacagtttc tttatggcag ggtgaaacgc aggtcgccag 2160
   cggcaccgcg cctttcggcg gtgaaattat cgatgagcgt ggtggttatg ccgatcgcgt 2220
   cacactacgt ctgaacgtcg aaaacccgaa actgtggagc gccgaaatcc cgaatctcta 2280
   tcgtgcggtg gttgaactgc acaccgccga cggcacgctg attgaagcag aagcctgcga 2340
   tgtcggtttc cgcgaggtgc ggattgaaaa tggtctgctg ctgctgaacg gcaagccgtt 2400
   gctgattcga ggcgttaacc gtcacgagca tcatcctctg catggtcagg tcatggatga 2460
   gcagacgatg gtgcaggata tcctgctgat gaagcagaac aactttaacg ccgtgcgctg 2520
   ttcgcattat ccgaaccatc cgctgtggta cacgctgtgc gaccgctacg gcctgtatgt 2580
   ggtggatgaa gccaatattg aaacccacgg catggtgcca atgaatcgtc tgaccgatga 2640
   tccgcgctgg ctaccggcga tgagcgaacg cgtaacgcga atggtgcagc gcgatcgtaa 2700
   tcacccgagt gtgatcatct ggtcgctggg gaatgaatca ggccacggcg ctaatcacga 2760
   cgcgctgtat cgctggatca aatctgtcga tccttcccgc ccggtgcagt atgaaggcgg 2820
   cggagccgac accacggcca ccgatattat ttgcccgatg tacgcgcgcg tggatgaaga 2880
   ccagcccttc ccggctgtgc cgaaatggtc catcaaaaaa tggctttcgc tacctggaga 2940
   gacgcgcccg ctgatccttt gcgaatacgc ccacgcgatg ggtaacagtc ttggcggttt 3000
   cgctaaatac tggcaggcgt ttcgtcagta tccccgttta cagggcggct tcgtctggga 3060
   ctgggtggat cagtcgctga ttaaatatga tgaaaacggc aacccgtggt cggcttacgg 3120
   cggtgatttt ggcgatacgc cgaacgatcg ccagttctgt atgaacggtc tggtctttgc 3180
   cgaccgcacg ccgcatccag cgctgacgga agcaaaacac cagcagcagt ttttccagtt 3240
   ccgtttatcc gggcaaacca tcgaagtgac cagcgaatac ctgttccgtc atagcgataa 3300
   cgagctcctg cactggatgg tggcgctgga tggtaagccg ctggcaagcg gtgaagtgcc 3360
   tctggatgtc gctccacaag gtaaacagtt gattgaactg cctgaactac cgcagccgga 3420
   gagcgccggg caactctggc tcacagtacg cgtagtgcaa ccgaacgcga ccgcatggtc 3480
   agaagccggg cacatcagcg cctggcagca gtggcgtctg gcggaaaacc tcagtgtgac 3540
   gctccccgcc gcgtcccacg ccatcccgca tctgaccacc agcgaaatgg atttttgcat 3600
   cgagctgggt aataagcgtt ggcaatttaa ccgccagtca ggctttcttt cacagatgtg 3660
   gattggcgat aaaaaacaac tgctgacgcc gctgcgcgat cagttcaccc gtgcaccgct 3720
   ggataacgac attggcgtaa gtgaagcgac ccgcattgac cctaacgcct gggtcgaacg 3780
   ctggaaggcg gcgggccatt accaggccga agcagcgttg ttgcagtgca cggcagatac 3840
   acttgctgat gcggtgctga ttacgaccgc tcacgcgtgg cagcatcagg ggaaaacctt 3900
   atttatcagc cggaaaacct accggattga tggtagtggt caaatggcga ttaccgttga 3960
   tgttgaagtg gcgagcgata caccgcatcc ggcgcggatt ggcctgaact gccagctggc 4020
   gcaggtagca gagcgggtaa actggctcgg attagggccg caagaaaact atcccgaccg 4080
   ccttactgcc gcctgttttg accgctggga tctgccattg tcagacatgt ataccccgta 4140
   cgtcttcccg agcgaaaacg gtctgcgctg cgggacgcgc gaattgaatt atggcccaca 4200
   ccagtggcgc ggcgacttcc agttcaacat cagccgctac agtcaacagc aactgatgga 4260
   aaccagccat cgccatctgc tgcacgcgga agaaggcaca tggctgaata tcgacggttt 4320
   ccatatgggg attggtggcg acgactcctg gagcccgtca gtatcggcgg aattacagct 4380
   gagcgccggt cgctaccatt accagttggt ctggtgtcaa aaataataat aaccgggcag 4440
   gccatgtctg cccgtatttc gcgtaaggaa atccattatg tactatttaa aaaacacaaa 4500
   cttttggatg ttcggtttat tctttttctt ttactttttt atcatgggag cctacttccc 4560
   gtttttcccg atttggctac atgacatcaa ccatatcagc aaaagtgata cgggtattat 4620
   ttttgccgct atttctctgt tctcgctatt attccaaccg ctgtttggtc tgctttctga 4680
   caaactcggc ctcgactcta ggcggccgcg gggatccaga catgataaga tacattgatg 4740
   agtttggaca aaccacaact agaatgcagt gaaaaaaatg ctttatttgt gaaatttgtg 4800
   atgctattgc tttatttgta accattataa gctgcaataa acaagttaac aacaacaatt 4860
   gcattcattt tatgtttcag gttcaggggg aggtgtggga ggttttttcg gatcctctag 4920
   agtcgagggc tgcagatctg tagggcgcag tagtccaggg tttccttgat gatgtcatac 4980
   ttatcctgtc cctttttttt ccacagctcg cggttgagga caaactcttc gcggtctttc 5040
   cagtggggat cgacggtatc gataagctgg ccgctctagt ggccgtacgg gcccacctgc 5100
   cgggccactt aattaaattt aaatcacgtg ctagcgctta agcttgaagt tcctattccg 5160
   aagttcctat tctctagaaa gtataggaac ttcggcgcgc cgtcgacgtt taaacatgca 5220
   tgaagttcct attccgaagt tcctattctc tagaaagtat aggaacttca taaaacctgc 5280
   aggcatgcaa gcgatcgcgg ccggccaagg cccgcggggc cactagaaga tgggcgggag 5340
   tcttctgggc aggcttaaag gctaacctgg tgtgtgggcg ttgtcctgca ggggaattga 5400
   acaggtgtaa aattggaggg acaagacttc ccacagattt tcggttttgt cgggaagttt 5460
   tttaataggg gcaaataagg aaaatgggag gataggtagt catctggggt tttatgcagc 5520
   aaaactacag gttattattg cttgtgatcc gcctcggagt attttccatc gaggtagatt 5580
   aaagacatgc tcacccgagt tttatactct cctgcttgag atccttacta cagtatgaaa 5640
   ttacagtgtc gcgagttaga ctatgtaagc agaattttaa tcatttttaa agagcccagt 5700
   acttcatatc catttctccc gctccttctg cagccttatc aaaaggtatt ttagaacact 5760
   cattttagcc ccattttcat ttattatact ggcttatcca acccctagac agagcattgg 5820
   cattttccct ttcctgatct tagaagtctg atgactcatg aaaccagaca gattagttac 5880
   atacaccaca aatcgaggct gtagctgggg cctcaacact gcagttcttt tataactcct 5940
   tagtacactt tttgttgatc ctttgccttg atccttaatt ttcagtgtct atcacctctc 6000
   ccgtcaggtg gtgttccaca tttgggccta ttctcagtcc agggagtttt acaacaatag 6060
   atgtattgag aatccaacct aaagcttaac tttccactcc catgaatgcc tctctccttt 6120
   ttctccattt ataaactgag ctattaacca ttaatggttt ccaggtggat gtctcctccc 6180
   ccaatattac ctgatgtatc ttacatattg ccaggctgat attttaagac attaaaaggt 6240
   atatttcatt attgagccac atggtattga ttactgctta ctaaaatttt gtcattgtac 6300
   acatctgtaa aaggtggttc cttttggaat gcaaagttca ggtgtttgtt gtctttcctg 6360
   acctaaggtc ttgtgagctt gtattttttc tatttaagca gtgctttctc ttggactggc 6420
   ttgactcatg gcattctaca cgttattgct ggtctaaatg tgattttgcc aagcttcttc 6480
   aggacctata attttgcttg acttgtagcc aaacacaagt aaaatgatta agcaacaaat 6540
   gtatttgtga agcttggttt ttaggttgtt gtgttgtgtg tgcttgtgct ctataataat 6600
   actatccagg ggctggagag gtggctcgga gttcaagagc acagactgct cttccagaag 6660
   tcctgagttc aattcccagc aaccacatgg tggctcacaa ccatctgtaa tgggatctga 6720
   tgccctcttc tggtgtgtct gaagaccaca agtgtattca cattaaataa ataaatcctc 6780
   cttcttcttc tttttttttt ttttaaagag aatactgtct ccagtagaat ttactgaagt 6840
   aatgaaatac tttgtgtttg ttccaatatg gtagccaata atcaaattac tctttaagca 6900
   ctggaaatgt taccaaggaa ctaattttta tttgaagtgt aactgtggac agaggagcca 6960
   taactgcaga cttgtgggat acagaagacc aatgcagact ttaatgtctt ttctcttaca 7020
   ctaagcaata aagaaataaa aattgaactt ctagtatcct atttgtttaa actgctagct 7080
   ttacttaact tttgtgcttc atctatacaa agctgaaagc taagtctgca gccattacta 7140
   aacatgaaag caagtaatga taattttgga tttcaaaaat gtagggccag agtttagcca 7200
   gccagtggtg gtgcttgcct ttatgccttt aatcccagca ctctggaggc agagacaggc 7260
   agatctctga gtttgagccc agcctggtct acacatcaag ttctatctag gatagccagg 7320
   aatacacaca gaaaccctgt tggggagggg ggctctgaga tttcataaaa ttataattga 7380
   agcattccct aatgagccac tatggatgtg gctaaatccg tctacctttc tgatgagatt 7440
   tgggtattat tttttctgtc tctgctgttg gttgggtctt ttgacactgt gggctttctt 7500
   taaagcctcc ttcctgccat gtggtctctt gtttgctact aacttcccat ggcttaaatg 7560
   gcatggcttt ttgccttcta agggcagctg ctgagatttg cagcctgatt tccagggtgg 7620
   ggttgggaaa tctttcaaac actaaaattg tcctttaatt ttttttttaa aaaatgggtt 7680
   atataataaa cctcataaaa tagttatgag gagtgaggtg gactaatatt aaatgagtcc 7740
   ctcccctata aaagagctat taaggctttt tgtcttatac ttaacttttt ttttaaatgt 7800
   ggtatcttta gaaccaaggg tcttagagtt ttagtataca gaaactgttg catcgcttaa 7860
   tcagattttc tagtttcaaa tccagagaat ccaaattctt cacagccaaa gtcaaattaa 7920
   gaatttctga cttttaatgt taatttgctt actgtgaata taaaaatgat agcttttcct 7980
   gaggcagggt ctcactatgt atctctgcct gatctgcaac aagatatgta gactaaagtt 8040
   ctgcctgctt ttgtctcctg aatactaagg ttaaaatgta gtaatacttt tggaacttgc 8100
   aggtcagatt cttttatagg ggacacacta agggagcttg ggtgatagtt ggtaaaatgt 8160
   gtttcaagtg atgaaaactt gaattattat caccgcaacc tactttttaa aaaaaaaagc 8220
   caggcctgtt agagcatgct taagggatcc ctaggacttg ctgagcacac aagagtagtt 8280
   acttggcagg ctcctggtga gagcatattt caaaaaacaa ggcagacaac caagaaacta 8340
   cagttaaggt tacctgtctt taaaccatct gcatatacac agggatatta aaatattcca 8400
   aataatattt cattcaagtt ttcccccatc aaattgggac atggatttct ccggtgaata 8460
   ggcagagttg gaaactaaac aaatgttggt tttgtgattt gtgaaattgt tttcaagtga 8520
   tagttaaagc ccatgagata cagaacaaag ctgctatttc gaggtctctt ggtttatact 8580
   cagaagcact tctttgggtt tccctgcact atcctgatca tgtgctaggc ctaccttagg 8640
   ctgattgttg ttcaaataaa cttaagtttc ctgtcaggtg atgtcatatg atttcatata 8700
   tcaaggcaaa acatgttata tatgttaaac atttgtactt aatgtgaaag ttaggtcttt 8760
   gtgggtttga tttttaattt tcaaaacctg agctaaataa gtcattttta catgtcttac 8820
   atttggtgga attgtataat tgtggtttgc aggcaagact ctctgaccta gtaaccctac 8880
   ctatagagca ctttgctggg tcacaagtct aggagtcaag catttcacct tgaagttgag 8940
   acgttttgtt agtgtatact agtttatatg ttggaggaca tgtttatcca gaagatattc 9000
   aggactattt ttgactgggc taaggaattg attctgatta gcactgttag tgagcattga 9060
   gtggccttta ggcttgaatt ggagtcactt gtatatctca aataatgctg gcctttttta 9120
   aaaagccctt gttctttatc accctgtttt ctacataatt tttgttcaaa gaaatacttg 9180
   tttggatctc cttttgacaa caatagcatg ttttcaagcc atattttttt tccttttttt 9240
   tttttttttt ggtttttcga gacagggttt ctctgtatag ccctggctgt cctggaactc 9300
   actttgtaga ccaggctggc ctcgaactca gaaatccgcc tgcctctgcc tcctgagtgc 9360
   cgggattaaa ggcgtgcacc accacgcctg gctaagttgg atattttgtt atataactat 9420
   aaccaatact aactccactg ggtggatttt taattcagtc agtagtctta agtggtcttt 9480
   attggccctt cattaaaatc tactgttcac tctaacagag gctgttggta ctagtggcac 9540
   ttaagcaact tcctacggat atactagcag attaagggtc agggatagaa actagtctag 9600
   cgttttgtat acctaccagc tttatactac cttgttctga tagaaatatt tcaggacatc 9660
   tagcttatcg atccgtcgac ggtatcgata agcttgatat cgaattccag cttttgttcc 9720
   ctttagtgag ggttaattgc gcgcttggcg taatcatggt catagctgtt tcctgtgtga 9780
   aattgttatc cgctcacaat tccacacaac atacgagccg gaagcataaa gtgtaaagcc 9840
   tggggtgcct aatgagtgag ctaactcaca ttaattgcgt tgcgctcact gcccgctttc 9900
   cagtcgggaa acctgtcgtg ccagctgcat taatgaatcg gccaacgcgc ggggagaggc 9960
   ggtttgcgta ttgggcgctc ttccgcttcc tcgctcactg actcgctgcg ctcggtcgtt 10020
   cggctgcggc gagcggtatc agctcactca aaggcggtaa tacggttatc cacagaatca 10080
   ggggataacg caggaaagaa catgtgagca aaaggccagc aaaaggccag gaaccgtaaa 10140
   aaggccgcgt tgctggcgtt tttccatagg ctccgccccc ctgacgagca tcacaaaaat 10200
   cgacgctcaa gtcagaggtg gcgaaacccg acaggactat aaagatacca ggcgtttccc 10260
   cctggaagct ccctcgtgcg ctctcctgtt ccgaccctgc cgcttaccgg atacctgtcc 10320
   gcctttctcc cttcgggaag cgtggcgctt tctcatagct cacgctgtag gtatctcagt 10380
   tcggtgtagg tcgttcgctc caagctgggc tgtgtgcacg aaccccccgt tcagcccgac 10440
   cgctgcgcct tatccggtaa ctatcgtctt gagtccaacc cggtaagaca cgacttatcg 10500
   ccactggcag cagccactgg taacaggatt agcagagcga ggtatgtagg cggtgctaca 10560
   gagttcttga agtggtggcc taactacggc tacactagaa ggacagtatt tggtatctgc 10620
   gctctgctga agccagttac cttcggaaaa agagttggta gctcttgatc cggcaaacaa 10680
   accaccgctg gtagcggtgg tttttttgtt tgcaagcagc agattacgcg cagaaaaaaa 10740
   ggatctcaag aagatccttt gatcttttct acggggtctg acgctcagtg gaacgaaaac 10800
   tcacgttaag ggattttggt catgagatta tcaaaaagga tcttcaccta gatcctttta 10860
   aattaaaaat gaagttttaa atcaatctaa agtatatatg agtaaacttg gtctgacagt 10920
   taccaatgct taatcagtga ggcacctatc tcagcgatct gtctatttcg ttcatccata 10980
   gttgcctgac tccccgtcgt gtagataact acgatacggg agggcttacc atctggcccc 11040
   agtgctgcaa tgataccgcg agacccacgc tcaccggctc cagatttatc agcaataaac 11100
   cagccagccg gaagggccga gcgcagaagt ggtcctgcaa ctttatccgc ctccatccag 11160
   tctattaatt gttgccggga agctagagta agtagttcgc cagttaatag tttgcgcaac 11220
   gttgttgcca ttgctacagg catcgtggtg tcacgctcgt cgtttggtat ggcttcattc 11280
   agctccggtt cccaacgatc aaggcgagtt acatgatccc ccatgttgtg caaaaaagcg 11340
   gttagctcct tcggtcctcc gatcgttgtc agaagtaagt tggccgcagt gttatcactc 11400
   atggttatgg cagcactgca taattctctt actgtcatgc catccgtaag atgcttttct 11460
   gtgactggtg agtactcaac caagtcattc tgagaatagt gtatgcggcg accgagttgc 11520
   tcttgcccgg cgtcaatacg ggataatacc gcgccacata gcagaacttt aaaagtgctc 11580
   atcattggaa aacgttcttc ggggcgaaaa ctctcaagga tcttaccgct gttgagatcc 11640
   agttcgatgt aacccactcg tgcacccaac tgatcttcag catcttttac tttcaccagc 11700
   gtttctgggt gagcaaaaac aggaaggcaa aatgccgcaa aaaagggaat aagggcgaca 11760
   cggaaatgtt gaatactcat actcttcctt tttcaatatt attgaagcat ttatcagggt 11820
   tattgtctca tgagcggata catatttgaa tgtatttaga aaaataaaca aataggggtt 11880
   ccgcgcacat ttccccgaaa agtgccacct aaattgtaag cgttaatatt ttgttaaaat 11940
   tcgcgttaaa tttttgttaa atcagctcat tttttaacca ataggccgaa atcggcaaaa 12000
   tcccttataa atcaaaagaa tagaccgaga tagggttgag tgttgttcca gtttggaaca 12060
   agagtccact attaaagaac gtggactcca acgtcaaagg gcgaaaaacc gtctatcagg 12120
   gcgatggccc actacgtgaa ccatcaccct aatcaagttt tttggggtcg aggtgccgta 12180
   aagcactaaa tcggaaccct aaagggagcc cccgatttag agcttgacgg ggaaagccgg 12240
   cgaacgtggc gagaaaggaa gggaagaaag cgaaaggagc gggcgctagg gcgctggcaa 12300
   gtgtagcggt cacgctgcgc gtaaccacca cacccgccgc gcttaatgcg ccgctacagg 12360
   gcgcgtccca ttcgccattc aggctgcgca actgttggga agggcgatcg gtgcgggcct 12420
   cttcgctatt acgccagctg gcgaaagggg gatgtgctgc aaggcgatta agttgggtaa 12480
   cgccagggtt ttcccagtca cgacgttgta aaacgacggc cagtgagcgc gcgtaatacg 12540
   actcactata gggcgaattg gagct 12565
<210> 2
   <211> 36
   <212> DNA
   <213> artificial sequence
<220>
   <223> /note="Description of artificial sequence: ZFN recognition site within the murine Rosa26 locus"
<400> 2
   tgcaactcca gtctttctag aagatgggcg ggagtc 36
<210> 3
   <211> 9489
   <212> DNA
   <213> artificial sequence
<220>
   <223> /note="Description of artificial sequence: Vector pROSA26.3-3"
<400> 3
   caccgcatta ccctgttatc cctagcggca ggccctccga gcgtggtgga gccgttctgt 60
   gagacagccg ggtacgagtc gtgacgctgg aaggggcaag cgggtggtgg gcaggaatgc 120
   ggtccgccct gcagcaaccg gagggggagg gagaagggag cggaaaagtc tccaccggac 180
   gcggccatgg ctcggggggg ggggggcagc ggaggagcgc ttccggccga cgtctcgtcg 240
   ctgattggct tcttttcctc ccgccgtgtg tgaaaacaca aatggcgtgt tttggttggc 300
   gtaaggcgcc tgtcagttaa cggcagccgg agtgcgcagc cgccggcagc ctcgctctgc 360
   ccactgggtg gggcgggagg taggtggggt gaggcgagct ggacgtgcgg gcgcggtcgg 420
   cctctggcgg ggcgggggag gggagggagg gtcagcgaaa gtagctcgcg cgcgagcggc 480
   cgcccaccct ccccttcctc tgggggagtc gttttacccg ccgccggccg ggcctcgtcg 540
   tctgattggc tctcggggcc cagaaaactg gcccttgcca ttggctcgtg ttcgtgcaag 600
   ttgagtccat ccgccggcca gcgggggcgg cgaggaggcg ctcccaggtt ccggccctcc 660
   cctcggcccc gcgccgcaga gtctggccgc gcgcccctgc gcaacgtggc aggaagcgcg 720
   cgctgggggc ggggacgggc agtagggctg agcggctgcg gggcgggtgc aagcacgttt 780
   ccgacttgag ttgcctcaag aggggcgtgc tgagccagac ctccatcgcg cactccgggg 840
   agtggaggga aggagcgagg gctcagttgg gctgttttgg aggcaggaag cacttgctct 900
   cccaaagtcg ctctgagttg ttatcagtaa gggagctgca gtggagtagg cggggagaag 960
   gccgcaccct tctccggagg ggggagggga gtgttgcaat acctttctgg gagttctctg 1020
   ctgcctcctg gcttctgagg accgccctgg gcctgggaga atcccttccc cctcttccct 1080
   cgtgatctgc aactccagtc tttctaggcg cgcccgggct gcagatctgt agggcgcagt 1140
   agtccagggt ttccttgatg atgtcatact tatcctgtcc cttttttttc cacagctcgc 1200
   ggttgaggac aaactcttcg cggtctttcc agtggggatc gacggtatcg ataagctggc 1260
   cgctctagga tccaccatgg tgagcaaggg cgaggagctg ttcaccgggg tggtgcccat 1320
   cctggtcgag ctggacggcg acgtaaacgg ccacaagttc agcgtgtccg gcgagggcga 1380
   gggcgatgcc acctacggca agctgaccct gaagctgatc tgcaccaccg gcaagctgcc 1440
   cgtgccctgg cccaccctcg tgaccaccct gggctacggc ctgcagtgct tcgcccgcta 1500
   ccccgaccac atgaagcagc acgacttctt caagtccgcc atgcccgaag gctacgtcca 1560
   ggagcgcacc atcttcttca aggacgacgg caactacaag acccgcgccg aggtgaagtt 1620
   cgagggcgac accctggtga accgcatcga gctgaagggc atcgacttca aggaggacgg 1680
   caacatcctg gggcacaagc tggagtacaa ctacaacagc cacaacgtct atatcaccgc 1740
   cgacaagcag aagaacggca tcaaggccaa cttcaagatc cgccacaaca tcgaggacgg 1800
   cggcgtgcag ctcgccgacc actaccagca gaacaccccc atcggcgacg gccccgtgct 1860
   gctgcccgac aaccactacc tgagctacca gtccgccctg agcaaagacc ccaacgagaa 1920
   gcgcgatcac atggtcctgc tggagttcgt gaccgccgcc gggatcactc tcggcatgga 1980
   cgagctgtac aagtaagaat tcaaggcctc tcgagcctct agaactatag tgagtcgtat 2040
   tacgtagatc cagacatgat aagatacatt gatgagtttg gacaaaccac aactagaatg 2100
   cagtgaaaaa aatgctttat ttgtgaaatt tgtgatgcta ttgctttatt tgtaaccatt 2160
   ataagctgca ataaacaagt taacaacaac aattgcattc attttatgtt tcaggttcag 2220
   ggggaggtgt gggaggtttt ttaattcgcg gccctagaag atgggcggga gtcttctggg 2280
   caggcttaaa ggctaacctg gtgtgtgggc gttgtcctgc aggggaattg aacaggtgta 2340
   aaattggagg gacaagactt cccacagatt ttcggttttg tcgggaagtt ttttaatagg 2400
   ggcaaataag gaaaatggga ggataggtag tcatctgggg ttttatgcag caaaactaca 2460
   ggttattatt gcttgtgatc cgcctcggag tattttccat cgaggtagat taaagacatg   2520
   ctcacccgag ttttatactc tcctgcttga gatccttact acagtatgaa attacagtgt   2580
   cgcgagttag actatgtaag cagaatttta atcattttta aagagcccag tacttcatat   2640
   ccatttctcc cgctccttct gcagccttat caaaaggtat tttagaacac tcattttagc   2700
   cccattttca tttattatac tggcttatcc aacccctaga cagagcattg gcattttccc   2760
   tttcctgatc ttagaagtct gatgactcat gaaaccagac agattagtta catacaccac   2820
   aaatcgaggc tgtagctggg gcctcaacac tgcagttctt ttataactcc ttagtacact   2880
   ttttgttgat cctttgcctt gatccttaat tttcagtgtc tatcacctct cccgtcaggt   2940
   ggtgttccac atttgggcct attctcagtc cagggagttt tacaacaata gatgtattga   3000
   gaatccaacc taaagcttaa ctttccactc ccatgaatgc ctctctcctt tttctccatt   3060
   tataaactga gctattaacc attaatggtt tccaggtgga tgtctcctcc cccaatatta   3120
   cctgatgtat cttacatatt gccaggctga tattttaaga cattaaaagg tatatttcat   3180
   tattgagcca catggtattg attactgctt actaaaattt tgtcattgta cacatctgta   3240
   aaaggtggtt ccttttggaa tgcaaagttc aggtgtttgt tgtctttcct gacctaaggt   3300
   cttgtgagct tgtatttttt ctatttaagc agtgctttct cttggactgg cttgactcat   3360
   ggcattctac acgttattgc tggtctaaat gtgattttgc caagcttctt caggacctat   3420
   aattttgctt gacttgtagc caaacacaag taaaatgatt aagcaacaaa tgtatttgtg   3480
   aagcttggtt tttaggttgt tgtgttgtgt gtgcttgtgc tctataataa tactatccag   3540
   gggctggaga ggtggctcgg agttcaagag cacagactgc tcttccagaa gtcctgagtt   3600
   caattcccag caaccacatg gtggctcaca accatctgta atgggatctg atgccctctt   3660
   ctggtgtgtc tgaagaccac aagtgtattc acattaaata aataaatcct ccttcttctt   3720
   cttttttttt tttttaaaga gaatactgtc tccagtagaa tttactgaag taatgaaata   3780
   ctttgtgttt gttccaatat ggtagccaat aatcaaatta ctctttaagc actggaaatg   3840
   ttaccaagga actaattttt atttgaagtg taactgtgga cagaggagcc ataactgcag   3900
   acttgtggga tacagaagac caatgcagac tttaatgtct tttctcttac actaagcaat   3960
   aaagaaataa aaattgaact tctagtatcc tatttgttta aactgctagc tttacttaac   4020
   ttttgtgctt catctataca aagctgaaag ctaagtctgc agccattact aaacatgaaa   4080
   gcaagtaatg ataattttgg atttcaaaaa tgtagggcca gagtttagcc agccagtggt   4140
   ggtgcttgcc tttatgcctt taatcccagc actctggagg cagagacagg cagatctctg   4200
   agtttgagcc cagcctggtc tacacatcaa gttctatcta ggatagccag gaatacacac   4260
   agaaaccctg ttggggaggg gggctctgag atttcataaa attataattg aagcattccc   4320
   taatgagcca ctatggatgt ggctaaatcc gtctaccttt ctgatgagat ttgggtatta   4380
   ttttttctgt ctctgctgtt ggttgggtct tttgacactg tgggctttct ttaaagcctc   4440
   cttcctgcca tgtggtctct tgtttgctac taacttccca tggcttaaat ggcatggctt   4500
   tttgccttct aagggcagct gctgagattt gcagcctgat ttccagggtg gggttgggaa   4560
   atctttcaaa cactaaaatt gtcctttaat ttttttttta aaaaatgggt tatataataa   4620
   acctcataaa atagttatga ggagtgaggt ggactaatat taaatgagtc cctcccctat   4680
   aaaagagcta ttaaggcttt ttgtcttata cttaactttt tttttaaatg tggtatcttt   4740
   agaaccaagg gtcttagagt tttagtatac agaaactgtt gcatcgctta atcagatttt   4800
   ctagtttcaa atccagagaa tccaaattct tcacagccaa agtcaaatta agaatttctg   4860
   acttttaatg ttaatttgct tactgtgaat ataaaaatga tagcttttcc tgaggcaggg   4920
   tctcactatg tatctctgcc tgatctgcaa caagatatgt agactaaagt tctgcctgct   4980
   tttgtctcct gaatactaag gttaaaatgt agtaatactt ttggaacttg caggtcagat   5040
   tcttttatag gggacacact aagggagctt gggtgatagt tggtaaaatg tgtttcaagt   5100
   gatgaaaact tgaattatta tcaccgcaac ctacttttta aaaaaaaaag ccaggcctgt   5160
   tagagcatgc ttaagggatc cctaggactt gctgagcaca caagagtagt tacttggcag   5220
   gctcctggtg agagcatatt tcaaaaaaca aggcagacaa ccaagaaact acagttaagg   5280
   ttacctgtct ttaaaccatc tgcatataca cagggatatt aaaatattcc aaataatatt   5340
   tcattcaagt tttcccccat caaattggga catggatttc tccggtgaat aggcagagtt   5400
   ggaaactaaa caaatgttgg ttttgtgatt tgtgaaattg ttttcaagtg atagttaaag   5460
   cccatgagat acagaacaaa gctgctattt cgaggtctct tggtttatac tcagaagcac   5520
   ttctttgggt ttccctgcac tatcctgatc atgtgctagg cctaccttag gctgattgtt   5580
   gttcaaataa acttaagttt cctgtcaggt gatgtcatat gatttcatat atcaaggcaa   5640
   aacatgttat atatgttaaa catttgtact taatgtgaaa gttaggtctt tgtgggtttg   5700
   atttttaatt ttcaaaacct gagctaaata agtcattttt acatgtctta catttggtgg   5760
   aattgtataa ttgtggtttg caggcaagac tctctgacct agtaacccta cctatagagc   5820
   actttgctgg gtcacaagtc taggagtcaa gcatttcacc ttgaagttga gacgttttgt   5880
   tagtgtatac tagtttatat gttggaggac atgtttatcc agaagatatt caggactatt   5940
   tttgactggg ctaaggaatt gattctgatt agcactgtta gtgagcattg agtggccttt   6000
   aggcttgaat tggagtcact tgtatatctc aaataatgct ggcctttttt aaaaagccct   6060
   tgttctttat caccctgttt tctacataat ttttgttcaa agaaatactt gtttggatct   6120
   ccttttgaca acaatagcat gttttcaagc catatttttt ttcctttttt tttttttttt 6180
   tggtttttcg agacagggtt tctctgtata gccctggctg tcctggaact cactttgtag 6240
   accaggctgg cctcgaactc agaaatccgc ctgcctctgc ctcctgagtg ccgggattaa 6300
   aggcgtgcac caccacgcct ggctaagttg gatattttgt tatataacta taaccaatac 6360
   taactccact gggtggattt ttaattcagt cagtagtctt aagtggtctt tattggccct 6420
   tcattaaaat ctactgttca ctctaacaga ggctgttggt actagtggca cttaagcaac 6480
   ttcctacgga tatactagca gattaagggt cagggataga aactagtcta gcgttttgta 6540
   tacctaccag ctttatacta ccttgttctg atagaaatat ttcaggacat ctagcttatc 6600
   gataccgtcg acggtatcga taagcttgat ccagcttttg ttccctttag tgagggttaa 6660
   ttgcgcgctt ggcgtaatca tggtcatagc tgtttcctgt gtgaaattgt tatccgctca 6720
   caattccaca caacatacga gccggaagca taaagtgtaa agcctggggt gcctaatgag 6780
   tgagctaact cacattaatt gcgttgcgct cactgcccgc tttccagtcg ggaaacctgt 6840
   cgtgccagct gcattaatga atcggccaac gcgcggggag aggcggtttg cgtattgggc 6900
   gctcttccgc ttcctcgctc actgactcgc tgcgctcggt cgttcggctg cggcgagcgg 6960
   tatcagctca ctcaaaggcg gtaatacggt tatccacaga atcaggggat aacgcaggaa 7020
   agaacatgtg agcaaaaggc cagcaaaagg ccaggaaccg taaaaaggcc gcgttgctgg 7080
   cgtttttcca taggctccgc ccccctgacg agcatcacaa aaatcgacgc tcaagtcaga 7140
   ggtggcgaaa cccgacagga ctataaagat accaggcgtt tccccctgga agctccctcg 7200
   tgcgctctcc tgttccgacc ctgccgctta ccggatacct gtccgccttt ctcccttcgg 7260
   gaagcgtggc gctttctcat agctcacgct gtaggtatct cagttcggtg taggtcgttc 7320
   gctccaagct gggctgtgtg cacgaacccc ccgttcagcc cgaccgctgc gccttatccg 7380
   gtaactatcg tcttgagtcc aacccggtaa gacacgactt atcgccactg gcagcagcca 7440
   ctggtaacag gattagcaga gcgaggtatg taggcggtgc tacagagttc ttgaagtggt 7500
   ggcctaacta cggctacact agaaggacag tatttggtat ctgcgctctg ctgaagccag 7560
   ttaccttcgg aaaaagagtt ggtagctctt gatccggcaa acaaaccacc gctggtagcg 7620
   gtggtttttt tgtttgcaag cagcagatta cgcgcagaaa aaaaggatct caagaagatc 7680
   ctttgatctt ttctacgggg tctgacgctc agtggaacga aaactcacgt taagggattt 7740
   tggtcatgag attatcaaaa aggatcttca cctagatcct tttaaattaa aaatgaagtt 7800
   ttaaatcaat ctaaagtata tatgagtaaa cttggtctga cagttaccaa tgcttaatca 7860
   gtgaggcacc tatctcagcg atctgtctat ttcgttcatc catagttgcc tgactccccg 7920
   tcgtgtagat aactacgata cgggagggct taccatctgg ccccagtgct gcaatgatac 7980
   cgcgagaccc acgctcaccg gctccagatt tatcagcaat aaaccagcca gccggaaggg 8040
   ccgagcgcag aagtggtcct gcaactttat ccgcctccat ccagtctatt aattgttgcc 8100
   gggaagctag agtaagtagt tcgccagtta atagtttgcg caacgttgtt gccattgcta 8160
   caggcatcgt ggtgtcacgc tcgtcgtttg gtatggcttc attcagctcc ggttcccaac 8220
   gatcaaggcg agttacatga tcccccatgt tgtgcaaaaa agcggttagc tccttcggtc 8280
   ctccgatcgt tgtcagaagt aagttggccg cagtgttatc actcatggtt atggcagcac 8340
   tgcataattc tcttactgtc atgccatccg taagatgctt ttctgtgact ggtgagtact 8400
   caaccaagtc attctgagaa tagtgtatgc ggcgaccgag ttgctcttgc ccggcgtcaa 8460
   tacgggataa taccgcgcca catagcagaa ctttaaaagt gctcatcatt ggaaaacgtt 8520
   cttcggggcg aaaactctca aggatcttac cgctgttgag atccagttcg atgtaaccca 8580
   ctcgtgcacc caactgatct tcagcatctt ttactttcac cagcgtttct gggtgagcaa 8640
   aaacaggaag gcaaaatgcc gcaaaaaagg gaataagggc gacacggaaa tgttgaatac 8700
   tcatactctt cctttttcaa tattattgaa gcatttatca gggttattgt ctcatgagcg 8760
   gatacatatt tgaatgtatt tagaaaaata aacaaatagg ggttccgcgc acatttcccc 8820
   gaaaagtgcc acctaaattg taagcgttaa tattttgtta aaattcgcgt taaatttttg 8880
   ttaaatcagc tcatttttta accaataggc cgaaatcggc aaaatccctt ataaatcaaa 8940
   agaatagacc gagatagggt tgagtgttgt tccagtttgg aacaagagtc cactattaaa 9000
   gaacgtggac tccaacgtca aagggcgaaa aaccgtctat cagggcgatg gcccactacg 9060
   tgaaccatca ccctaatcaa gttttttggg gtcgaggtgc cgtaaagcac taaatcggaa 9120
   ccctaaaggg agcccccgat ttagagcttg acggggaaag ccggcgaacg tggcgagaaa 9180
   ggaagggaag aaagcgaaag gagcgggcgc tagggcgctg gcaagtgtag cggtcacgct 9240
   gcgcgtaacc accacacccg ccgcgcttaa tgcgccgcta cagggcgcgt cccattcgcc 9300
   attcaggctg cgcaactgtt gggaagggcg atcggtgcgg gcctcttcgc tattacgcca 9360
   gctggcgaaa gggggatgtg ctgcaaggcg attaagttgg gtaacgccag ggttttccca 9420
   gtcacgacgt tgtaaaacga cggccagtga gcgcgcgtaa tacgactcac tatagggcga 9480
   attggagct 9489
<210> 4
   <211> 460
   <212> DNA
   <213> artificial sequence
<220>
   <223> /note="Description of artificial sequence: Rosa26 5'-probe"
<400> 4
   gcccttcttc tcagctacct ttacacacca ttgcaccgct cttgcccaga gagaaaggct     60
   ctccttcatc tagtcgaccc cactaccttt ttaatgtctt ccctgggtca ggactcttcc    120
   cctcccccta ctctggtctc ccctttttgc ctgggtattg cctactccac gtttataccc   180
   ttttcaggag aggcctccca accctgctct caaaatacac atactttttt ttctgtccct    240
   gagcccccca cctcccctgt tcttgcggcc ttgtgacaac tctggtcgct cgtgggggcc    300
   cagtcctccc ctccataatc ttcctgaacg cctctcctct ggttttccag ttcctatctc    360
   agatggctgc tgcttttccc acaccaaaga cattaccttc gccaccccca cctcacattc   420
   ttggactccc tgtggcgtat gccccagtat ccttaagggc                          460
<210> 5
   <211> 660
   <212> DNA
   <213> artificial sequence
<220>
   <223> /note="Description of artificial sequence: Rosa26 3'-probe"
<400> 5
   acaaacactt ctacatgtca gttttatata tattataaca caaacggtgt taattgaata     60
   attaagcatt ttaaaagcat gaaactacaa ccattgttca taaataagtt ctaacgttgt    120
   ggttttataa tttggaactt tggggaaatg ttcaaaacat tttatgacaa acacctttat    180
   cctcacatac aaagaaagct acaatactta aaatggtaat tgcataacac attgcatgga    240
   ttatacaagg tgtaacttaa gagtttaatt catacagaac acatatgttc tacctacact    300
   acattagaaa aatccaaact gcatagcaac atttaacaca gtgacattac tgtcactgac    360
   catcatgcct ctgcttgctt ctgagaacat aaatggcaac atcttgggat tatgaacatc    420
   atgttgatct cgaagacctg ttgctgctca gacagcagaa atatagcctt aaacaagcac    480
   tgtcctgtcc tcaaggaatg atctaaagga ggaaaaacaa taattacaca attaatattc    540
   ttaattaaga ctttaagagc catggcaatg ttcaagcagg accaaatgtg gtgcagtgtt    600
   gagggcaatc tgggaaggtt ccttaagaag ttatgttctg agaccattct cagtggctca    660
<210> 6
   <211> 1250
   <212> DNA
   <213> artificial sequence
<220>
   <223> /note="Description of artificial sequence: beta-Galactosidase probe
<400> 6
   ggccgcaatt cccggggatc gaaagagcct gctaaagcaa aaaagaagtc accatgtcgt 60
   ttactttgac caacaagaac gtgattttcg ttgccggtct gggaggcatt ggtctggaca 120
   ccagcaagga gctgctcaag cgcgatcccg tcgttttaca acgtcgtgac tgggaaaacc 180
   ctggcgttac ccaacttaat cgccttgcag cacatccccc tttcgccagc tggcgtaata 240
   gcgaagaggc ccgcaccgat cgcccttccc aacagttgcg cagcctgaat ggcgaatggc 300
   gctttgcctg gtttccggca ccagaagcgg tgccggaaag ctggctggag tgcgatcttc 360
   ctgaggccga tactgtcgtc gtcccctcaa actggcagat gcacggttac gatgcgccca 420
   tctacaccaa cgtaacctat cccattacgg tcaatccgcc gtttgttccc acggagaatc 480
   cgacgggttg ttactcgctc acatttaatg ttgatgaaag ctggctacag gaaggccaga 540
   cgcgaattat ttttgatggc gttaactcgg cgtttcatct gtggtgcaac gggcgctggg 600
   tcggttacgg ccaggacagt cgtttgccgt ctgaatttga cctgagcgca tttttacgcg 660
   ccggagaaaa ccgcctcgcg gtgatggtgc tgcgttggag tgacggcagt tatctggaag 720
   atcaggatat gtggcggatg agcggcattt tccgtgacgt ctcgttgctg cataaaccga 780
   ctacacaaat cagcgatttc catgttgcca ctcgctttaa tgatgatttc agccgcgctg 840
   tactggaggc tgaagttcag atgtgcggcg agttgcgtga ctacctacgg gtaacagttt 900
   ctttatggca gggtgaaacg caggtcgcca gcggcaccgc gcctttcggc ggtgaaatta 960
   tcgatgagcg tggtggttat gccgatcgcg tcacactacg tctgaacgtc gaaaacccga 1020
   aactgtggag cgccgaaatc ccgaatctct atcgtgcggt ggttgaactg cacaccgccg 1080
   acggcacgct gattgaagca gaagcctgcg atgtcggttt ccgcgaggtg cggattgaaa 1140
   atggtctgct gctgctgaac ggcaagccgt tgctgattcg aggcgttaac cgtcacgagc 1200
   atcatcctct gcatggtcag gtcatggatg agcagacgat ggtgcaggat 1250
<210> 7
   <211> 730
   <212> DNA
   <213> artificial sequence
<220>
   <223> /note="Description of artificial sequence: Venus probe"
<400> 7
   gatccaccat ggtgagcaag ggcgaggagc tgttcaccgg ggtggtgccc atcctggtcg 60
   agctggacgg cgacgtaaac ggccacaagt tcagcgtgtc cggcgagggc gagggcgatg 120
   ccacctacgg caagctgacc ctgaagctga tctgcaccac cggcaagctg cccgtgccct    180
   ggcccaccct cgtgaccacc ctgggctacg gcctgcagtg cttcgcccgc taccccgacc    240
   acatgaagca gcacgacttc ttcaagtccg ccatgcccga aggctacgtc caggagcgca 300
   ccatcttctt caaggacgac ggcaactaca agacccgcgc cgaggtgaag ttcgagggcg 360
   acaccctggt gaaccgcatc gagctgaagg gcatcgactt caaggaggac ggcaacatcc 420
   tggggcacaa gctggagtac aactacaaca gccacaacgt ctatatcacc gccgacaagc 480
   agaagaacgg catcaaggcc aacttcaaga tccgccacaa catcgaggac ggcggcgtgc 540
   agctcgccga ccactaccag cagaacaccc ccatcggcga cggccccgtg ctgctgcccg 600
   acaaccacta cctgagctac cagtccgccc tgagcaaaga ccccaacgag aagcgcgatc 660
   acatggtcct gctggagttc gtgaccgccg ccgggatcac tctcggcatg gacgagctgt 720
   acaagtaaga 730
<210> 8
   <211> 21
   <212> DNA
   <213> artificial sequence
<220>
   <223> /note="Description of artificial sequence: Primer 5HA"
<400> 8
   aaagtcgctc tgagttgtta t 21
<210> 9
   <211> 20
   <212> DNA
   <213> artificial sequence
<220>
   <223> /note="Description of artificial sequence: Primer SA1"
<400> 9
   cgatccccag tactggaaag 20
<210> 10
   <211> 20
   <212> DNA
   <213> artificial sequence
<220>
   <223> /note="Description of artificial sequence: Primer SA2"
<400> 10
   ttgctttagc aggctctttc 20
<210> 11
   <211> 23
   <212> DNA
   <213> artificial sequence
<220>
   <223> /note="Description of artificial sequence: Rosa 3HA"
<400> 11
   cacaccaggt tagcctttaa gcc 23
<210> 12
   <211> 21
   <212> DNA
   <213> artificial sequence
<220>
   <223> /note="Description of artificial sequence: Primer pA1"
<400> 12
   tcggatcctc tagagtcgag g 21
<210> 13
   <211> 22
   <212> DNA
   <213> artificial sequence
<220>
   <223> /note="Description of artificial sequence: Primer pA2"
<400> 13
   aaaccacaac tagaatgcag tg 22
<210> 14
   <211> 23
   <212> DNA
   <213> artificial sequence
<220>
   <223> /note="Description of artificial sequence: Primer pA3"
<400> 14
   aagatacatt gatgagtttg gac 23
<210> 15
   <211> 24
   <212> DNA
   <213> artificial sequence
<220>
   <223> /note="Description of artificial sequence: Primer SA3"
<400> 15
   tttccagtgg ggatcgacgg tatc 24

## Description

The present invention relates to a method of modifying a target sequence in the genome of a non-human mammalian or avian oocyte by homologous recombination with a donor nucleic acid sequence, the method comprising the steps (a) introducing into the oocyte a zinc finger nuclease or a nucleic add molecule encoding the zinc finger nuclease, in expressible form, wherein the zinc finger nuclease specifically binds within the target sequence and introduces a double strand break within the target sequence; and (b) introducing a nucleic acid molecule into the oocyte, wherein the nucleic add molecule comprises the donor nucleic acid sequence and regions homologous to the target sequence. The present invention further relates to a method of producing a non-human mammal or an avian carrying a modified target sequence in its genome.

With the complete elucidation of the human, mouse and other mammalian genome sequences a major challenge is the functional characterization of every gene within the genome and the identification of gene products and their molecular interaction network In the past two decades the mouse has developed into the prime mammalian genetic model to study human biology and disease because methods are available that allow the production of targeted, predesigned mouse mutants. This reverse genetics approach that enables the production of germ line and conditional knockout mice by gene targeting, relies on the use of murine embryonic stem (ES) cell lines. ES cell lines exhibit unique properties such that they are able, once established from the inner cell mass of a mouse blastocyst, to renew indefinitely in cell culture while retaining their early pluripotent differentiation state. This property allows to grow ES cells in large numbers and, since most mutagenesis methods are inefficient, to select rare genetic variants that are expanded into a pure stem cell clone that harbours a specific genetic alteration in the target gene. Upon introduction of ES cells into mouse blastocysts and subsequent embryo transfer these cells contribute to all cell types of the developing chimaeric embryo, including the germ line. By mating of germ line chimaeras to normal mice a genetic modification engineered in ES cells is inherited to their offspring and thereby transferred into the mouse germ line.

The basis for reverse mouse genetics was initially established in the decade of 1980-90 in three steps and the basic scheme that is followed since that time is essentially unchanged. The first of these steps was the establishment of ES cell lines from cultured murine blastocysts and of culture conditions that maintain their pluripotent differentiation state *in vitro* (Evans MJ, Kaufman MH., Nature 1981; 292:154-6; Martin GR. Proc Natl Acad Sci U S A 1981; 78:7634-8). A few years later it was first reported that ES cells, upon microinjection into blastocysts, are able to colonize the germ line in chimaeric mice (Bradley A, Evans M, Kaufman MH, Robertson E., Nature 1984; 309:255-6; Gossler A, Doetschman T, Korn R, Serfling E, Kemler R., Proc Natl Acad Sci U S A 1986; 83:9065-9). The third step concerns the technology to introduce pre-planned, inactivating mutations into target genes in ES cells by homologous recombination between a gene targeting vector and endogenous loci (gene targeting). Gene targeting allows the introduction of pre-designed, site-specific modifications into the mouse genome (Capecchi MR. Trends Genet 1989; 5:70-6). Since the first demonstration of homologous recombination in ES cells in 1987 (Thomas KR, Capecchi MR., Cell 1987; 51:503-12) and the establishment of the first knockout mouse strain in 1989 (Schwartzberg PL, Goff SP, Robertson EJ., Science 1989; 246:799-803) gene targeting was adopted to many other genes and has been used in the last decades to generate more than 3000 knockout mouse strains that provided a wealth of information on in vivo gene functions (Collins FS, Rossant J, Wurst W., Cell 2007; 128:9-13; Capecchi, M. R., Nat Rev Genet 2005; 6: 507-12).

Targeted gene inactivation in ES cells can be achieved through the insertion of a selectable marker (mostly the neomycin phosphotransferase gene, neo) into an exon of the target gene or the replacement of one or more exons. The mutant allele is initially assembled in a specifically designed gene targeting vector such that the selectable marker is flanked at both sides with genomic segments of the target gene that serve as homology regions to initiate homologous recombination. The frequency of homologous recombination increases with the length of these homology arms. Usually arms with a combined length of 10-15 kb are cloned into standard, high copy plasmid vectors that accommodate up to 20 kb of foreign DNA. To select against random vector integrations a negative selectable marker, such as the *Herpes simplex* thymidine kinase or diphtheria toxin gene, can be included at one end of the targeting vector. Upon electroporation of such a vector into ES cells and the selection of stable integrants, clones that underwent a homologous recombination event can be identified through the analysis of genomic DNA using a PCR or Southern blot strategy. Using such standard gene targeting vectors the efficiency at which homologous recombinant ES cell clones are obtained is the range of 0.1% to 10% as compared to the number of stable transfected (Neo resistant) ES cell clones. This rate depends on the length of the vector homology region, the degree of sequence identity of this region with the genomic DNA of the ES cell line and likely on the differential accessibility of individual genomic loci to homologous recombination. Optimal rates are achieved with longer homology regions and by the use of genomic fragments that exhibit sequence identity to the genome of the ES cell line, i.e. both should be isogenic and derived from the same inbred mouse strain (te Riele H, Maandag ER, Berns A. 1992. Proc Natl Acad Sci U S A 89:5128-5132). Since the frequency of stable transfection of ES cells by electroporation is about 10⁻⁴ (i.e. 1 Neo resistant cone from 10.000 electroporated cells), the absolute efficiency of obtaining homologous recombinant ES cells falls in the range of 10⁻⁵ - 10⁻⁷ (Cheah SS, Behringer RR., Methods Mol Biol 2000; 136: 455-63; DeChiara TM.; Methods Mol Biol 2001; 158: 19-45; Hasty P, Abuin A, Bradley A., 2000, In Gene Targeting: a practical approach, ed. AL Joyner, pp. 1-35. Oxford: Oxford University Press; Nagy A, Gertsenstein M, Vintersten K, Behringer R., 2003. Manipulating the Mouse Embryo. Cold Spring Harbour, New York: Cold Spring Harbour Laboratory Press)

Upon the isolation of recombinant ES cell clones modified ES cells are injected into blastocysts to transmit the mutant allele through the germ line of chimaeras and to establish a mutant strain. Through interbreeding of heterozygous mutants homozygotes are obtained that can be used for phenotype analysis.

Using the "classical" gene targeting approach described above germ line mutants are obtained that harbour the knockout mutation in all cells throughout development. This strategy identifies the first essential function of a gene during ontogeny. If the gene product fulfils an important role in development its inactivation can lead to embryonic lethality precluding further analysis in adult mice. In general about 30% of all knockout mouse strains exhibit an embryonic lethal phenotype, for specific classes of genes, e.g. those regulating angiogenesis, this rate can reach 100%. To avoid embryonic lethality and to study gene function only in specific cell types Gu et al. (Gu H, Marth JD, Orban PC, Mossmann H, Rajewsky K., Science 1994; 265:103-6) introduced a modified, conditional gene targeting scheme that allows to restrict gene inactivation to specific cell types or developmental stages. In a conditional mutant, gene inactivation is achieved by the insertion of two 34 bp recognition (IoxP) sites of the site-specific DNA recombinase Cre into introns of the target gene such that recombination results in the deletion of IoxP-flanked exons. Conditional mutants initially require the generation of two mouse strains: one strain harbouring a IoxP flanked gene segment obtained by gene targeting in ES cells and a second, transgenic strain expressing Cre recombinase in one or several cell types. The conditional mutant is generated by crossing these two strains such that target gene inactivation occurs in a spatial and temporal restricted manner, according to the pattern of recombinase expression in the Cre transgenic strain (Nagy A, Gertsenstein M, Vintersten K, Behringer R. 2003. Manipulating the Mouse Embryo, third edition ed. Cold Spring Harbour, New York: Cold Spring Harbour Laboratory Press; Torres RM, Kühn R. 1997. Laboratory protocols for conditional gene targeting. Oxford: Oxford University Press). Conditional mutants have been used to address various biological questions which could not be resolved with germ line mutants, often because a null allele results in an embryonic or neonatal lethal phenotype.

Taken together, gene targeting in ES cells has revolutionised the *in vivo* analysis of mammalian gene function using the mouse as genetic model system. However, since germ line competent ES cell lines that can be genetically modified could be established only from mice, this reverse genetics approach is presently restricted to this rodent species. The exception from this rule is achieved by homologous recombination in primary cells from pig and sheep followed by the transplantation of nuclei from recombined somatic cells into enucleated oocytes (cloning) (Lai L, Prather RS. 2003. Reprod Biol Endocrinol 2003; 1:82; Gong M, Rong YS. 2003. Curr Opin Genet Dev 13:215-220). Since this methodology is inefficient and time consuming it has not the potential to develop into a simple routine procedure.

Although the generation of targeted mouse mutants via genome engineering in ES cells and the derivation of germ line transmitting chimaeras is established as a routine procedure this approach typically requires 1-2 years of hands on work for vector construction, ES cell culture and selection and the breeding of chimaeras. Typical problems that are encountered during a gene targeting project are the low efficiency of homologous recombination in ES cells and the loss of the germ line competence of ES cells during the long *in vitro* culture and selection phase. Therefore, the successful generation of even a single line of knockout mice requires considerable time, the combined efforts of specialists in molecular biology, ES cell culture and embryo manipulation, and the associated technical infrastructure.

Experiments in model systems have demonstrated that the frequency of homologous recombination of a gene targeting vector is strongly increased if a double-strand break is induced within its chromosomal target sequence. Using the yeast homing endonuclease I-Scel, that cuts DNA at an 18 base pair-long recognition site, it was initially shown that homologous recombination and gene targeting are stimulated over 1000-fold in mammalian cells when a recognition site is inserted into a target gene and I-Scel is expressed in these cells (Rouet, P., Smih, F., Jasin, M.; Mol Cell Biol 1994; 14: 8096-8106; Rouet, P., Smih, F. Jasin, M.; Proc Natl Acad Sci USA 1994; 91: 6064-6068).

Zinc finger nucleases (ZFNs) were developed as a method to apply the stimulatory power of double strand breaks to sequences of endogenous genes, without the need to introduce an artificial nuclease recognition site. Zinc finger nucleases are fusion proteins that link a zinc finger DNA binding domain to a non-specific nuclease domain (Porteus MH, Carroll D.; Nat Biotechnol 2005; 23:967-973; Durai S, Mani M, Kandavelou K, Wu J, Porteus MH, Chandrasegaran S. 2005. Nucleic Acids Res 2005; 33:5978-5990). For this purpose the non-sequence-specific cleavage domain of the Fok-I restriction endonuclease (Fn domain) can be fused to a zinc finger type DNA binding domain. Zinc finger protein modules occur in the DNA-binding domain of the most abundant family of eukaryotic transcription factors. Each finger is composed of 30 amino-acids, folds into a ββα configuration that binds one Zn+ atom by two cysteines and two histidine residues, and contacts 3 base pairs of DNA. To recognize novel target sequences individual zinc fingers with different triplet targets can be combined and by altering individual amino acid residues the specificity for an individual finger can be altered. Individual fingers have been designed to recognize many of the 64 different target triplets, but the greatest success has been in designing zinc fingers to recognize 5'-GNN-3'triplets.

It is currently believed that the dimerisation of the nuclease domain is required for the cleavage of DNA by a zinc finger nuclease. Thus, a single three-finger zinc finger nuclease that recognises a 9 bp sequence can act as homodimer to cleave a genomic target that contains an inverted repeat of the recognition sequence, separated by a spacer of 6 bp. Target sequences consisting of a unique 18 bp sequence can be approached by the use of two different three finger zinc finger nucleases that act as heterodimer, each recognising a 9 bp sequence. The induction of a double-stranded break into a target locus of interest strongly stimulates the cellular mechanisms for DNA repair. The activation of these repair functions can be exploited in two ways to induce different types of gene mutations.

Upon expression of a zinc finger nuclease, in the absence of a gene targeting vector for homology directed repair, the cells frequently close the double-strand break by non-homologous end-joining (NHEJ). This mechanism is error-prone and frequently leads to the deletion or insertion of multiple nucleotides at the cleavage site. If the ZFN target site is located within the coding region of a gene it is thereby possible to identify and select mutants that exhibit reading frameshift mutations from a mutagenised population and that represent non-functional knockout alleles of the targeted gene. By this application knockout alleles were generated in mammalian cell lines and knockout zebra fish and rats were obtained upon the expression of ZFN mRNA in one cell embryos (Santiago Y, Chan E, Liu PQ, Orlando S, Zhang L, Urnov FD, Holmes MC, Guschin D, Waite A, Miller JC, Rebar EJ, Gregory PD, Klug A, Collingwood TN.; Proc Natl Acad Sci U S A 2008; 105:5809-5814; Doyon Y, McCammon JM, Miller JC, Faraji F, Ngo C, Katibah GE, Amora R, Hocking TD, Zhang L, Rebar EJ, Gregory PD, Urnov FD, Amacher SL.; Nat Biotechnol 2008; 26:702-708; Geurts AM, Cost GJ, Freyvert Y, Zeitler B, Miller JC, Choi VM, Jenkins SS, Wood A, Cui X, Meng X, Vincent A, Lam S, Michalkiewicz M, Schilling R, Foeckler J, Kalloway S, Weiler H, Menoret S, Anegon I, Davis GD, Zhang L, Rebar EJ, Gregory PD, Urnov FD, Jacob HJ, Buelow R.; Science 2009; 325:433).

Upon expression of a zinc finger nuclease in the presence of an exogeneous gene targeting vector that contains homology regions to the target gene, the vector can be used as a template for homology driven repair of the double strand break through gene conversion. Usually gene targeting with ZFNs is induced at the site of the double-strand break but also occurs in a region of several hundred base pairs aside the break. The frequency of this homologous recombination event is several orders of magnitude higher as usually obtained by the use of a gene targeting vector in the absence of a zinc finger nuclease. This methodology has been applied to gene engineering in mammalian cell lines and gene correction in primary human cells (Urnov FD, Miller JC, Lee YL, Beausejour CM, Rock JM, Augustus S, Jamieson AC, Porteus MH, Gregory PD, Holmes MC.; Nature 2005; 435:646-651; Porteus MH, Baltimore D. 2003. Science 300:763; Hockemeyer D, Soldner F, Beard C, Gao Q, Mitalipova M, DeKelver RC, Katibah GE, Amora R, Boydston EA, Zeitler B, Meng X, Miller JC, Zhang L, Rebar EJ, Gregory PD, Urnov FD, Jaenisch R.; Nat Biotechnol 2009; 27:851-857).

Although the use of zinc finger nucleases results in a higher frequency of homologous recombination, the generation of targeted mouse mutants via genome engineering in ES cells and the derivation of germ line transmitting chimaeras still requires considerable time and effort. Due to the lack of embryonic stem cells lines, other mammalian species are not amenable to these methods at all.

WO 03/087341 describes methods for the genetic manipulation of drosophila and Arabidopsis, including the stable integration of zinc finger nuclease constructs as well as donor DNA into the genome of the host cell of interest and activation of the expression by exposure to a heat shock.

While genetically engineered mice serve as research tool, other mammalian species and also birds, in particular chicken have great potential in animal biotechnology for the production of recombinant proteins. Transgenesis in chicken has been achieved by the inflection of early embryos with viral vectors (Ivarie R.; Trends Biotechnol 2006; 24:99-101; Kamihira M, Nishijima K, lijima S.; Adv Biochem Eng Biotechnol 2004; 91:171-189). The targeted modification of endogenous genes, however, has not been described for birds.

The technical problem underlying the present invention is thus the provision of improved means and methods for modifying the mammalian or avian genome.

The solution to this technical problem is achieved by providing the embodiments characterised in the claims.

Accordingly, the present invention relates to a method of modifying a target sequence in the genome of a non-human mammalian or avian oocyte by homologous recombination with a donor nucleic acid sequence, the method comprising the steps (a) introducing into the oocyte a zinc finger nuclease or a nucleic acid molecule encoding the zinc finger nuclease in expressible form, wherein the zinc finger nuclease specifically binds within the target sequence and introduces a double strand break within the target sequence; and (b) introducing a nucleic acid molecule into the oocyte, wherein the nucleic acid molecule comprises the donor nucleic acid sequence and regions homologous to the target sequence.

The term "modifying" as used in accordance with the present invention refers to site-specific genomic manipulations resulting in changes in the nucleotide sequence. The genetic material comprising these changes in its nucleotide sequence is also referred to herein as the "modified target sequence". The term "modifying" includes, but is not limited to, substitution, insertion and deletion of one or more nucleotides within the target sequence.

The term "substitution", as used herein, refers to the replacement of nucleotides with other nucleotides. The term includes for example the replacement of single nucleotides resulting in point mutations. Said point mutations can lead to an amino acid exchange in the resulting protein product but may also not be reflected on the amino acid level. Also encompassed by the term "substitution" are mutations resulting in the replacement of multiple nucleotides, such as for example parts of genes, such as parts of exons or introns as well as replacement of entire genes.

The term "insertion" in accordance with the present invention refers to the incorporation of one or more nucleotides into a nucleic acid molecule. Insertion of parts of genes, such as parts of exons or introns as well as insertion of entire genes is also encompassed by the term "insertion". When the number of inserted nucleotides is not dividable by three, the insertion can result in a frameshift mutation within a coding sequence of a gene. Such frameshift mutations will alter the amino acids encoded by a gene following the mutation. In some cases, such a mutation will cause the active translation of the gene to encounter a premature stop codon, resulting in an end to translation and the production of a truncated protein. When the number of inserted nucleotides is instead dividable by three, the resulting insertion is an "in-frame insertion". In this case, the reading frame remains intact after the insertion and translation will most likely run to completion if the inserted nucleotides do not code for a stop codon. However, because of the inserted nucleotides, the finished protein will contain, depending on the size of the insertion, one or multiple new amino acids that may effect the function of the protein.

The term "deletion" as used in accordance with the present invention refers to the loss of nucleotides or part of genes, such as exons or introns as well as entire genes. As defined with regard to the term "insertion", the deletion of a number of nucleotides that is not evenly dividable by three will lead to a frameshift mutation, causing all of the codons occurring after the deletion to be read incorrectly during translation, potentially producing a severely altered and most likely nonfunctional protein. If a deletion does not result in a frameshift mutation, i.e. because the number of nucleotides deleted is dividable by three, the resulting protein is nonetheless altered as the finished protein will lack, depending on the size of the deletion, several amino acids that may effect the function of the protein.

The above defined modifications are not restricted to coding regions in the genome, but can also occur in non-coding regions of the target genome, for example in regulatory regions such as promoter or enhancer elements or in introns.

Examples of modifications of the target genome include, without being limited, the introduction of mutations into a wildtype gene in order to analyse its effect on gene function; the replacement of an entire gene with a mutated gene or, alternatively, if the target sequence comprises mutation(s), the alteration of these mutations to identify which one is causative of a particular effect; the removal of entire genes or proteins or the removal of regulatory elements from genes or proteins as well as the introduction of fusion-partners, such as for example purification tags such as the his-tag or the tap-tag etc.

In accordance with the present invention, the term "target sequence in the genome" refers to the genomic location that is to be modified by the method of the invention. The "target sequence in the genome" comprises but is not restricted to the nucleotide(s) subject to the particular modification and the sequence to which the regions homologous to the target sequence bind. In other words, the term "target sequence in the genome" also comprises the sequence surrounding the relevant nucleotide(s) to be modified. Preferably, the term "target sequence" refers to the entire gene to be modified.

The term "oocyte", as used herein, refers to the female germ cell involved in reproduction, i.e. the ovum or egg cell. In accordance with the present invention, the term "oocyte" comprises both oocytes before fertilisation as well as fertilised oocytes, which are also called zygotes. Thus, the oocyte before fertilisation comprises only maternal chromosomes, whereas an oocyte after fertilisation comprises both maternal and paternal chromosomes. After fertilisation, the oocyte remains in a double-haploid status for several hours, in mice for example for up to 18 hours after fertilisation.

The term "homologous recombination", is used according to the definitions provided in the art. Thus, it refers to a mechanism of genetic recombination in which two DNA strands comprising similar nucleotide sequences exchange genetic material. Cells use homologous recombination during meiosis, where it serves to rearrange DNA to create an entirely unique set of haploid chromosomes, but also for the repair of damaged DNA, in particular for the repair of double strand breaks. The mechanism of homologous recombination is well known to the skilled person and has been described, for example by Paques and Haber (Paques F, Haber JE.; Microbiol Mol Biol Rev 1999; 63:349-404)

In accordance with the present invention, the term "donor nucleic acid sequence" refers to a nucleic acid sequence that serves as a template in the process of homologous recombination and that carries the modification that is to be introduced into the target sequence. By using this donor nucleic acid sequence as a template, the genetic information, including the modifications, is copied into the target sequence within the genome of the oocyte. In non-limiting examples, the donor nucleic acid sequence can be essentially identical to the part of the target sequence to be replaced, with the exception of one nucleotide which differs and results in the introduction of a point mutation upon homologous recombination or it can consist of an additional gene previously not present in the target sequence.

The term "zinc finger nuclease", as used in accordance with the present invention, refers to a fusion protein consisting of the non-specific cleavage domain of an endonuclease, preferably a restriction endonuclease, and a DNA-binding domain consisting of zinc finger motifs. The term "zinc finger nuclease", as used herein, refers to a functional zinc finger nuclease that essentially retains the enzymatic activity of the (restriction) endonuclease.

It is presently believed that the mechanism of double-strand cleavage by zinc finger nuclease requires dimerisation of the nuclease domain in order to cut the DNA substrate (Durai *et al.* 2005). Dimerisation of zinc finger nucleases can result in the formation of homodimers if only one type of zinc finger nuclease is present or in the formation of heterodimers, when different types of zinc finger nucleases are present. It is preferred in accordance with the present invention that at least two different types of zinc finger nucleases having differing zinc finger motifs are introduced into the oocyte. The at least two different types of zinc finger nucleases can be introduced into the oocyte either separately or together. Also envisaged herein is a zinc finger nuclease, which is provided as a functional dimer via linkage of two subunits of identical or different zinc finger nucleases prior to introduction into the oocyte.

In accordance with the present invention, (restriction) endonuclease function is essentially retained if at least 60% of the biological activity of the endonuclease activity are retained. Preferably, at least 75% or at least 80% of the endonuclease activity are retained. More preferred is that at least 90% such as at least 95%, even more preferred at least 98% such as at least 99% of the biological activity of the endonuclease are retained. Most preferred is that the biological activity is fully, i.e. to 100%, retained. Also in accordance with the invention are zinc finger nucleases having increased biological activity compared to the endogenous endonuclease, i.e. more than 100% activity. Methods of assessing biological activity of (restriction) endonucleases are well known to the person skilled in the art and include, without being limiting, the incubation of an endonuclease with recombinant DNA and the analysis of the reaction products by gel electrophoresis (Bloch KD.; Curr Protoc Mol Biol 2001; Chapter 3:Unit 3.2).

The term "nucleic acid molecule encoding the zinc finger nuclease in expressible form" refers to a nucleic acid molecule which, upon expression in a cell or a cell-free system, results in a functional zinc finger nuclease. Nucleic acid molecules as well as nucleic acid sequences, as used throughout the present description, include DNA, such as cDNA or genomic DNA, and RNA. Preferably, embodiments reciting "RNA" are directed to mRNA. Furthermore included is genomic RNA, such as in case of RNA of RNA viruses.

It will be readily appreciated by the skilled person that more than one nucleic acid molecule may encode a zinc finger nuclease in accordance with the present invention due to the degeneracy of the genetic code. Degeneracy results because a triplet code designates 20 amino acids and a stop codon. Because four bases exist which are utilized to encode genetic information, triplet codons are required to produce at least 21 different codes. The possible 4³ possibilities for bases in triplets give 64 possible codons, meaning that some degeneracy must exist. As a result, some amino acids are encoded by more than one triplet, i.e. by up to six. The degeneracy mostly arises from alterations in the third position in a triplet. This means that nucleic acid molecules having different sequences, but still encoding the same zinc finger nuclease can be employed in accordance with the present invention.

The terms "endonuclease" and "restriction endonuclease" are used herein according to the well-known definitions provided by the art. Both terms thus refer to enzymes capable of cutting nucleic acids by cleaving the phosphodiester bond within a polynucleotide chain. Preferably, the endonuclease is a type II S restriction endonuclease, such as for example Fokl, AlwI, SfaNI, SapI, Plel, NmeAIII, Mboll, MlyI, Mmel, HpYAV, HphI, Hgal, Faul, Earl, EciI, BtgZI, CspCI, BspQI, BspMI, BsaXI, BsgI, Bsel, BpuEIBmrIBcgIBbvI, Bael, BbsIAlwI, or AcuI or a type III restriction endonuclease (e.g. EcoP1I, EcoP15I, HinfIII). More preferably, the endonuclease is FokI endonuclease. FokI is a bacterial type IIS restriction endonuclease. It recognises the non-palindromic penta-deoxyribonucleotide 5'-GGATG-3': 5'-CATCC-3' in duplex DNA and cleaves 9/13 nucleotides downstream of the recognition site. Fokl does not recognise any specific-sequence at the site of cleavage. Once the DNA-binding domain (either of the naturally occurring endonuclease, e.g. FokI or, in accordance with the present invention, of the zinc finger nuclease) is anchored at the recognition site, a signal is transmitted to the endonuclease domain and cleavage occurs. The distance of the cleavage site to the DNA-binding site of the zinc finger nuclease depends on the particular endonuclease present in the zinc finger nuclease. For example, the zinc finger nucleases employed in the examples of the present invention cleaves in the middle of a 6 bp sequence that is flanked by the two binding sites of the zinc finger proteins. As a further example, naturally occurring endonucleases such as FokI and EcoP15I cut at 9/13 and 27 bp distance from the DNA binding site, respectively.

Zinc finger motifs - also referred to herein as C₂H₂ zinc fingers - bind DNA by inserting their α-helix into the major groove of the DNA double helix. Each zinc finger motif primarily binds to a triplet within the DNA substrate. Binding to longer DNA sequences is achieved by linking several of these zinc finger motifs in tandem to form zinc finger proteins. The use of zinc finger proteins for the creation of zinc finger nucleases that recognize and cleave a specific target sequence depends on the reliable creation of zinc finger proteins that can specifically recognize said particular target. Methods for the generation of specific zinc finger nucleases are known to the skilled person and have been described, for example in Durai *et al.* 2005.

In accordance with the present invention, the term "specifically binds within the target sequence and introduces a double strand break within the target sequence" means that the zinc finger nuclease is designed such that statistically it only binds to a particular sequence and does not bind to an unrelated sequence elsewhere in the genome. Preferably, the zinc finger nuclease comprises at least four zinc finger motifs. In the case of zinc finger nucleases consisting of dimers as described above this means that each monomer of the zinc finger nuclease comprises at least two zinc finger motifs. More preferably, the zinc finger nuclease comprises at least six zinc finger motifs, such as for example at least eight or at least ten zinc finger motifs. Methods for testing the DNA-binding specificity of a zinc finger nuclease are known to the skilled person and include, without being limiting, phage display selection methods and the bacterial two hybrid system (Durai S, Mani M, Kandavelou K, Wu J, Porteus MH, Chandrasegaran S. 2005. Nucleic Acids Res 2005; 33:5978-5990).

Preferably, the binding site of the zinc finger nuclease is up to 500 nucleotides, such as up to 250 nucleotides, up to 100 nucleotides, up to 50 nucleotides, up to 25 nucleotides, up to 10 nucleotides such as up to 5 nucleotides upstream (i.e. 5') or downstream (i.e. 3') of the nucleotide(s) that is/are modified in accordance with the present invention.

In accordance with the method of modifying a target sequence of the present invention, the nucleic acid molecule introduced into the oocyte in step (b) comprises the donor nucleic acid sequence as defined above as well as additional regions that are homologous to the target sequence.

The term "regions homologous to the target sequence" (also referred to as "homology arms" herein), in accordance with the present invention, refers to regions having sufficient sequence identity to ensure specific binding to the target sequence. Methods to evaluate the identity level between two nucleic acid sequences are well known in the art. For example, the sequences can be aligned electronically using suitable computer programs known in the art. Such programs comprise BLAST (Altschul et al. (1990) J. Mol. Biol. 215, 403), variants thereof such as WU-BLAST (Altschul and Gish (1996) Methods Enzymol. 266, 460), FASTA (Pearson and Lipman (1988) Proc. Natl. Acad. Sci. USA 85, 2444) or implementations of the Smith-Waterman algorithm (SSEARCH, Smith and Waterman (1981) J. Mol. Biol., 147, 195). These programs, in addition to providing a pairwise sequence alignment, also report the sequence identity level (usually in percent identity) and the probability for the occurrence of the alignment by chance (P-value).

Preferably, the "regions homologous to the target sequence" have a sequence identity with the corresponding part of the target sequence of at least 95%, more preferred at least 97%, more preferred at least 98%, more preferred at least 99%, even more preferred at least 99.9% and most preferred 100%. The above defined sequence identities are defined only with respect to those parts of the target sequence which serve as binding sites for the homology arms. Thus, the overall sequence identity between the entire target sequence and the homologous regions of the nucleic acid molecule of step (b) of the method of modifying a target sequence of the present invention can differ from the above defined sequence identities, due to the presence of the part of the target sequence which is to be replaced by the donor nucleic acid sequence.

It is preferred that at least two regions homologous to the target sequence are present in the nucleic acid molecule of (b).

In accordance with the method of the present invention, the steps of introducing a zinc finger nuclease into the oocyte and of introducing a nucleic acid molecule into the oocyte are either carried out concomitantly, i.e. at the same time or are carried out separately, i.e. individually and at different time points. When the steps are carried out concomitantly, both the zinc finger nuclease and the nucleic acid molecule can be administered in parallel, for example using two separate injection needles or can be mixed together and, for example, be injected using one needle.

In accordance with the present invention it was surprisingly found that it is possible to introduce targeted gene modifications into the genome of mammalian and avian zygotes and to achieve an unexpectedly high frequency of homologous recombination of up to 47%.

The often cited dictum "omne vivum ex ovo" by the ovulist W. Harvey (Exercitationies de generatione animalium; London 1651), to date refers to one of the most complex cell transformations in biology, the re-modelling of a fertilised oocyte into a totipotent zygote. Remarkably, this transition occurs in the absence of transcription factors and therefore depends on mRNAs accumulated in the oocyte during oogenesis. A growing mouse oocyte, arrested at diplotene of its first meiotic prophase, transcribes and translates many of its own genes, thereby producing a store of proteins sufficient to support development up to the 8-cell stage. These transcripts guide oocytes on the two steps of oocyte maturation and egg activation to become zygotes. Typically, oocytes are ovulated and become competent for fertilisation before reaching a second arrest point. When an oocyte matures into an egg, it arrests in metaphase of its second meiotic division where transcription stops and translation of mRNA is reduced. At this point an ovulated mouse egg has a diameter of 0.085 mm, with a volume of ∼ 300 picoliter it exceeds 1000-fold the size of a typical somatic cell (Nagy A, Gertsenstein M, Vintersten K, Behringer R., 2003. Manipulating the Mouse Embryo. Cold Spring Harbour, New York: Cold Spring Harbour Laboratory Press).
Life and the embryonic development of a mammal begin when sperm fertilises an egg to form a zygote. Fertilization of the egg triggers egg activation to complete the transformation to a zygote by signaling the completion of meiosis and the formation of pronuclei. At this stage the zygote represents a 1-cell embryo that contains a haploid paternal pronucleus derived from the sperm and a haploid maternal pronucleus derived from the oocyte. In mice this totipotent single cell stage lasts for only ∼ 18 hours until the first mitotic division occurs.

As totipotent single entities, mammalian zygotes could be regarded as a preferred substrate for genome engineering since the germ line of the entire animal is accessible within a single cell. However, the experimental accessibility and manipulation of zygotes is severely restricted by the very limited numbers at which they are available (dozens-hundred) and their very short lasting nature. These parameters readily explain that the vast majority of genome manipulations, that occur at frequencies of below 10⁻⁵ like gene targeting, can be successfully performed only in cultured embryonic stem cells that are grown up to a number of 10⁷ cells in a single standard culture plate. The only exception from this rule concerns the generation of transgenic mice by pronuclear DNA injection that has been developed into a routine procedure due to the high frequency of transgene integration in up to 30% of injected zygotes (Palmiter RD, Brinster RL.; Annu Rev Genet 1986; 20:465-499). Since microinjected transgenes randomly integrate into the genome, this method can only be used to express additional genes on the background of an otherwise normal genome, but does not allow the targeted modification of endogenous genes.

An early report to characterise the potential of zygotes for targeted gene manipulation by Brinster (Brinster RL, Braun RE, Lo D, Avarbock MR, Oram F, Palmiter RD.; Proc Natl Acad Sci U S A 1989; 86:7087-7091), showed that this approach is not practical as only one targeted mouse was obtained from > 10.000 zygotes within 14 months of injections. Thus, Brinster *et al.* discouraged any further attempts in this direction. In addition to a low recombination frequency, Brinster *et al.* noted a high number of spontaneously occurring, undesired mutations within the targeted allele that severely compromised the function of the (repaired) histocompatibility class II gene. From the experience of Brinster *et al.* it could be extrapolated that the physiological, biochemical and epigenetic context of genomic DNA in the zygotic pronuclei are unfavourable to achieve targeted genetic manipulations, except for the random integration of transgenes that occurs at high frequency.

In addition, the biology of oocyte development into an embryo provides further obstacles for targeted genetic manipulations. In fertilized mammalian eggs, the two pronuclei that undergo DNA replication, do not fuse directly but approach each other and remain distinct until the membrane of each pronucleus has broken down in preparation for the zygote's first mitotic division that produces a 2-cell embryo. The 1-cell zygote stage is characterised by unique transcriptional and translation control mechanisms. One of the most striking features is a time-dependent mechanism, referred to as the zygotic clock, that delays the expression of the zygotic genome for ∼24 h after fertilization, regardless of whether or not the one-cell embryo has completed S phase and formed a two-cell embryo (Nothias JY, Majumder S, Kaneko KJ, DePamphilis ML.; J Biol Chem 1995;270:22077-22080). In nature, the zygotic clock provides the advantage of delaying zygotic gene activation (ZGA) until chromatin can be remodelled from a condensed meiotic state to one in which selected genes can be transcribed. Since the paternal genome is completely packaged with protamines that must be replaced with histones, some genes might be prematurely expressed if ZGA were not prevented. Cell-specific transcription requires that newly minted zygotic chromosomes repress most, if not all, promoters until development progresses to a stage where specific promoters can be activated by specific enhancers or trans-activators. In the mouse, formation of a 2-cell embryo marks the transition from maternal gene dependence to zygotic gene activation (ZGA). Among mammals, the extent of development prior to zygotic gene activation (ZGA) varies among species from one to four cleavage events. Maternal mRNA degradation is triggered by meiotic maturation and 90% completed in 2-cell embryos, although maternal protein synthesis continues into the 8-cell stage. In addition to transcriptional control, the zygotic clock delays the translation of nascent mRNA until the 2-cell stage (Nothias JY, Miranda M, DePamphilis ML.; EMBO J 1996; 15:5715-5725). Therefore, the production of proteins from transgenic expression vectors injected into pronuclei is not achieved until 10 - 12 hours after the appearance of mRNA.

Geurts *et al.* have decently found that zinc finger nucleases can be used to induce double strand breaks In the genome of rat zygotes (Geurts AM, Cost GJ, Freyvert Y, Zeitler B, Miller JC, Choi VM, Jenkins SS, Wood A, Cui X, Meng X, Vincent A, Lam S, Michalkiewicz M, Schilling R, Foeckler J, Kalloway S, Weiler H, Menoret S, Anegon I, Davis GD, Zhang L, Rebar EJ, Gregory PD, Urnov FD, Jacob HJ, Buelow R.; Science 2009; 325:433). In this work the induced strand breaks were left for the endogenous, error prone DNA repair mechanism in order to later identify randomly occurring mutant alleles that lost or acquired nucleotides at the site of DNA cleavage. Provided that the zinc finger nuclease cleavage site is located within an exon region of a gene, a reading frame shift will occur in some of the mutant alleles and thereby lead to the production of truncated, non-functional protein. However, this method only leads to the generation of undirected mutations within the coding region of a gene. So far, it has not been possible to induce directed modifications like pre-planned nucleotide substitutions, to insert exogeneous DNA sequences like reporter genes and recombinase recognition sites or to replace e.g. murine versus human coding regions.

To introduce such genetic modifications requires homologous recombination of a specifically designed gene targeting vector with a target gene. Since procedures to achieve high rate homologous recombination in zygotes were not known so far, gene targeting in somatic cells and the subsequent nuclear transfer into enucleated oocytes from sheep and pig have been used as a surrogate technique (Lai L, Prather RS. 2003. Reprod Biol Endocrinol 2003; 1:82; Gong M, Rong YS. 2003. Curr Opin Genet Dev 13:215-220). However, both techniques are demanding and not very efficient and their combined use is impractical and not well suited for routine application.

In accordance with the present invention a method of introducing genetic modifications into a target genome is provided that overcomes the above discussed problems currently faced by the skilled person. Using the method of the present invention it is now possible to generate genetically modified animals faster, easier and more cost-effective than using any of the prior art methods.

In an alternative embodiment, the present invention relates to a method of modifying a target sequence in the genome of a non-human mammalian or avian oocyte by homologous recombination with a donor nucleic acid sequence, the method comprising the steps (a) introducing a fusion protein comprising a DNA-binding domain and a non-specific cleavage domain of a restriction nuclease into the oocyte, wherein the fusion protein specifically binds within the target sequence and introduces a double strand break within the target sequence; and (b) introducing a nucleic acid molecule into the oocyte, wherein the nucleic acid molecule comprises the donor nucleic acid sequence and regions homologous to the target sequence.

Preferably, the DNA-binding domain is selected from the group consisting of helix-loop-helix binding proteins, basic leucine zipper (bZip) proteins or the classes of single or triple zinc finger proteins.

The family of helix-loop-helix binding proteins comprises standard helix-turn-helix binding proteins such as the *Escherichia coli* lactose repressor or the tryptophan repressor, homeodomain-proteins such as the *Drosophila* Antennapedia protein, paired homeodomain proteins such as the Vertebrate Pax transcription factors, POU domain proteins such as the Vertebrate regulatory proteins PIT-1, OCT-1 and OCT-2, winged helix-turn-holix proteins such as the GABP regulatory protein of higher eukaryotes as well as high mobility group (HMG) domain proteins.

The bZip family consist of a basic region which interacts with the major groove of a DNA molecule through hydrogen bonding, and a hydrophobic leucine zipper region which is responsible for dimerisation. Non-rimiting examples of bZip proteins are c-fos and c-jun.

The family of single zinc finger proteins comprises for example transcription regulating proteins like GAGA, while the family of triple zinc finger proteins comprises for example transcription regulating proteins like Krox24, Egr1, BKLF and SP1.

In a preferred embodiment of the method of the invention, the non-human mammalian or avian oocyte is a fertilised mammalian or avian oocyte.

As defined above, the term fertilised oocyte" refers to an oocyte after fusion with the fertilizing sperm. For a period of many hours (such as up to 18 hours in mice) after fertilisation, the oocyte is in a double-haploid state, comprising one maternal haploid pronucleus and one paternal haploid pronucleus. After migration of the two pronuclei together, their membranes break down, and the two genomes condense into chromosomes, thereby reconstituting a diploid organism. Preferably, the mammalian or avian oocyte used in the method of the present invention is a fertilised mammalian or avian oocyte in the double-haploid state.

In another preferred embodiment of the method of the invention, the zinc finger nuclease and the nucleic acid molecule are introduced into the oocyte by microinjection.

Microinjection into the oocyte can be carried out by injection into the nucleus (before fertilisation), the pronucleus (after fertilisation) and/or by injection into the cytoplasm (both before and after fertilisation). When a fertilised oocyte is employed, injection into the pronucleus is carried out either for one pronucleus or for both pronuclei. Injection of the zinc finger nuclease or of a DNA encoding the zinc finger nuclease of step (a) of the method of modifying a target sequence of the present invention is preferably into the nucleus/pronucleus, while injection of an mRNA encoding the zinc finger nuclease of step (a) is preferably into the cytoplasm. Injection of the nucleic acid molecule of step (b) is preferably into the nucleus/pronucleus. However, injection of the nucleic acid molecule of step (b) can also be carried out into the cytoplasm when said nucleic acid molecule is provided as a nucleic acid sequence having a nuclear localisation signal to ensure delivery into the nucleus/pronucleus. Preferably, the microinjection is carried out by injection into both the nucleus/pronucleus and the cytoplasm. For example, the needle can be introduced into the nucleus/pronucleus and a first amount of the zinc finger nuclease and/or nucleic acid molecule are injected into the nucleus/pronucleus. While removing the needle from the oocyte, a second amount of the zinc finger nuclease and/or nucleic acid molecule is injected into the cytoplasm.

Methods for carrying out microinjection are well known in the art and are described for example in Nagy et al. (Nagy A, Gertsenstein M, Vintersten K, Behringer R., 2003. Manipulating the Mouse Embryo. Cold Spring Harbour, New York: Cold Spring Harbour Laboratory Press) as well as in the examples herein below.

In a more preferred embodiment, the nucleic acid molecule encoding the zinc finger nuclease in expressible form is mRNA.

In another preferred embodiment of the method of the invention, the regions homologous to the target sequence are localised at the 5' and 3' end of the donor nucleic acid sequence.

In this preferred embodiment, the donor nucleic acid sequence is flanked by the two regions homologous to the target sequence such that the nucleic acid molecule used in the method of the present invention consists of a first region homologous to the target sequence, followed by the donor nucleic add sequence and then a second region homologous to the target sequence.

In a further preferred embodiment of the method of the invention, the regions homologous to the target sequence comprised in the nucleic acid molecule have a length of at least 400 bp each. More preferably, the regions each have a length of at least 500 nucleotides, such as at least 600 nucleotides, at least 750 bp nucleotides, more preferably at least 1000 nucleotides, such as at least 1500 nucleotides, even more preferably at least 2000 nucleotides and most preferably at least 2500 nucleotides. The maximum length of the regions homologous to the target sequence comprised in the nucleic acid molecule depends on the type of cloning vector used and can be up to a length 20.000 nucleotides each in E. coli high copy plasmids using the col EI replication origin (e.g. pBluescript) or up to a length of 300.000 nucleotides each in plasmids-using the F-factor origin (e.g. in BAC vectors such as for example pTARBAC1).

In a further preferred embodiment of the method of the invention, the modification of the target sequence is selected from the group consisting of substitution, insertion and deletion of at least one nucleotide of the target sequence. Preferred in accordance with the present invention are substitutions, for example substitutions of 1 to 3 nucleotides and insertions of exogenous sequences, such as IoxP sites (34 nucleotides long) or cDNAs, such as for example for reporter genes. Such cDNAs for reporter genes can, for example, be up to 6 kb long.

In another preferred embodiment of the method of the invention, the non-human oocyte is from a mammal selected from the group consisting of rodents, dogs, felides, monkeys, rabbits, pigs, or cows or wherein the oocyte is from an avian selected from the group consisting of chickens, turkeys, pheasants, ducks, geese, quails and ratites including ostriches, emus and cassowaries.

All of the mammals and avians described herein are well known to the skilled person and are taxonomically defined in accordance with the prior art and the common general knowledge of the skilled person.

Non-limiting examples of "rodents" are mice, rats, squirrels, chipmunks, gophers, porcupines, beavers, hamsters, gerbils, guinea pigs, degus, chinchillas, prairie dogs, and groundhogs. Non-limiting examples of "dogs" include members of the subspecies *canis lupus familiaris* as well as wolves, foxes, jackals, and coyotes.

Non-limiting examples of "felides" include members of the two subfamilles: the pantherinae, including lions, tigers, Jaguars and leopards and the felinae, including cougars, cheetahs, servals, lynxes, caracals, ocelots and domestic cats.

The term "primates", as used herein, refers to all monkey including for example cercopithecold (old world monkey) or platyrrhine (new world monkey) as well as lemurs, tarsiers, apes and marmosets (Callithrix jacchus).

In accordance with the present invention, the mammalian oocyte is not a human oocyte. Also in accordance with the present invention the fertilized oocyte is not a human oocyte.

The present invention further relates to a method of producing a non-human mammal or an avian carrying a modified target sequence in its genome, the method comprising (a) modifying the target sequence in the genome of a mammalian or avian oocyte in accordance with the method of the invention; (b) transferring the oocyte obtained in (a) to a non-human pseudopregnant female host; and (c) analysing the offspring delivered by the female host for the presence of the modification.

In accordance with the present invention, the term "transferring the oocyte obtained in (a) to a pseudopregnant female host" includes the transfer of the fertilised oocyte but also the transfer of pre-implantation embryos of for example the 2-cell, 4-cell, 8-cell, 16-cell and blastocyst (70-to 100-cell) stage. Thus, for the method of producing a non-human mammal or an avian, fertilisation of the oocyte is required. Said fertilisation can occur before the modification of the target sequence in step (a) in accordance with the method of producing a non-human mammal or an avian of the invention, i.e. a fertilised oocyte can be used for the method of modifying a target sequence in accordance with the invention. The fertilisation can also be carried out after the modification of the target sequence in step (a), i.e. a non-fertilised oocyte can be used for the method of modifying a target sequence in accordance with the invention, wherein the oocyte is subsequently fertilised before transfer into the pseudopregnant female host. The fertilised oocyte can be directly transferred to the pseudopregnant female host after carrying out the method of modifying the target sequence in the genome. Alternatively, the oocyte can be kept in culture to develop to the above mentioned stages of development before transfer to the female host. Methods for transferring the oocyte to a pseudopregnant female host are well known in the art and are, for example, described in Nagy et al., (Nagy A, Gertsenstein M, Vintersten K, Behringer R., 2003. Manipulating the Mouse Embryo. Cold Spring Harbour, New York: Cold Spring Harbour Laboratory Press).

The step of analysing in (c) for the presence of the modification in the offspring delivered by the female host provides the necessary information whether or not the produced non-human mammal or the avian carries the modified target sequence in its genome. Thus, the presence of the modification is indicative of said offspring carrying a modified target sequence in its genome whereas the absence of the modification is indicative of said offspring not carrying the modified target sequence in its genome. Methods for analysing for the presence or absence of a modification are well known in the art and include, without being limiting, assays based on physical separation of nucleic acid molecules, sequencing assays as well as cleavage and digestion assays and DNA analysis by the polymerase chain reaction (PCR).

Examples for assays based on physical separation of nucleic acid molecules include without limitation MALDI-TOF, denaturating gradient gel electrophoresis and other such methods known in the art, see for example Petersen et al., Hum. Mutat. 20 (2002) 253-259; Hsia et al., Theor. Appl. Genet. 111 (2005) 218-225; Tost and Gut, Clin. Biochem. 35 (2005) 335-350; Palais et al., Anal. Biochem. 346 (2005) 167-175.

Examples for sequencing assays comprise without limitation approaches of sequence analysis by direct sequencing, fluorescent SSCP in an automated DNA sequencer and Pyrosequencing. These procedures are common in the art, see e.g. Adams et al. (Ed.), "Automated DNA Sequencing and Analysis", Academic Press, 1994; Alphey, "DNA Sequencing: From Experimental Methods to Bioinformatics", Springer Verlag Publishing, 1997; Ramon et al., J. Transl. Med. 1 (2003) 9; Meng et al., J. Clin. Endocrinol. Metab. 90 (2005) 3419-3422.

Examples for cleavage and digestion assays include without limitation restriction digestion assays such as restriction fragments length polymorphism assays (RFLP assays), RNase protection assays, assays based on chemical cleavage methods and enzyme mismatch cleavage assays, see e.g. Youil et al., Proc. Natl. Acad. Sci. U.S.A. 92 (1995) 87-91; Todd et al., J. Oral Maxil. Surg. 59 (2001) 660-667; Amar et al., J. Clin. Microbiol. 40 (2002) 446-452.

It is further envisaged in accordance with the method of producing a non-human mammal or an avian carrying a modified target sequence in its genome that the step of analysis of successful genomic modification is carried out before transplantation into the female host. As a non-limiting example, the oocyte can be cultured to the 2-cell, 4-cell or 8-cell stage and one cell can be removed without destroying or altering the resulting embryo. Analysis for the genomic constitution, e.g. the presence or absence of the genomic modification, can then be carried out using for example PCR or southern blotting techniques. Such methods of analysis of successful genotyping prior to transplantation are known in the art and are described, for example Peippo et al. (Peippo J, Viitala S, Virta J, Raty M, Tammiranta N, Lamminen T, Aro J, Myllymaki H, Vilkki J.; Mol Reprod Dev 2007; 74:1373-1378).

The non-human mammal or avian produced by the method of the invention is, inter alia, useful to study the function of genes of interest and the phenotypic expression/outcome of modifications of the genome in such animals. It is furthermore envisaged, that the non-human mammals of the invention can be employed as disease models and for testing therapeutic agents/compositions. Furthermore, the non-human mammals of the invention can also be used for livestock breeding.

In a preferred embodiment, the non-human mammal is selected from the group consisting of rodents, dogs, felides, primates, rabbits, pigs, or cows or wherein the oocyte is from an avian selected from the group consisting of chickens, turkeys, pheasants, ducks, geese, quails and ratites including ostriches, emus and cassowaries.

The figures show:

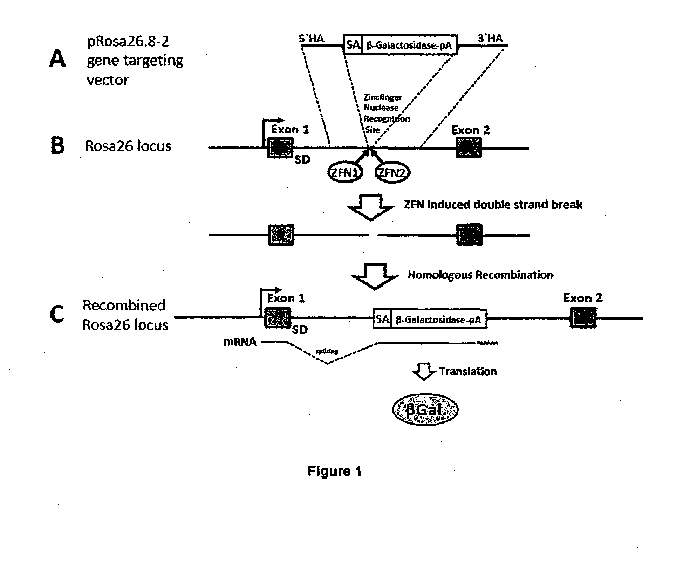

**Figure 1****.** Gene targeting vector pRosa26.8-2, structure of the Rosa26 locus and recognition sequence for zincfinger nuclease fusion proteins.
   A: Structure of the gene targeting vector pRosa26.8-2. The 5' and 3' homology regions (5'HR, 3'HR) to the Rosa26 locus are flanking the reporter gene cassette comprising a splice acceptor (SA) sequence, the β-gatactosidase coding region and a polyadenylation sequence (pA); B: Structure of the genomic Rosa26 locus. Shown are the first 2 exons of Rosa26 and the Rosa26 promoter (arrow) upstream of exon 1. The homology regions to the pRosa26.8-2 vector within intron 1 are indicated by stippled lines and the target site for the pair of zincfinger nucleases is shown by an arrow. Upon occurrence of a double strand break at this site homologous recombination ist stimulated resulting in the recombined Rosa26 locus; C: Recombined Rosa26 locus. Upon insertion of the reporter gene cassette into the zincfinger nuclease target site the reporters splice acceptor is spliced to the Rosa26 exon 1 sequence, leading to the production of a mRNA coding for β-galactosidase (βGal.).

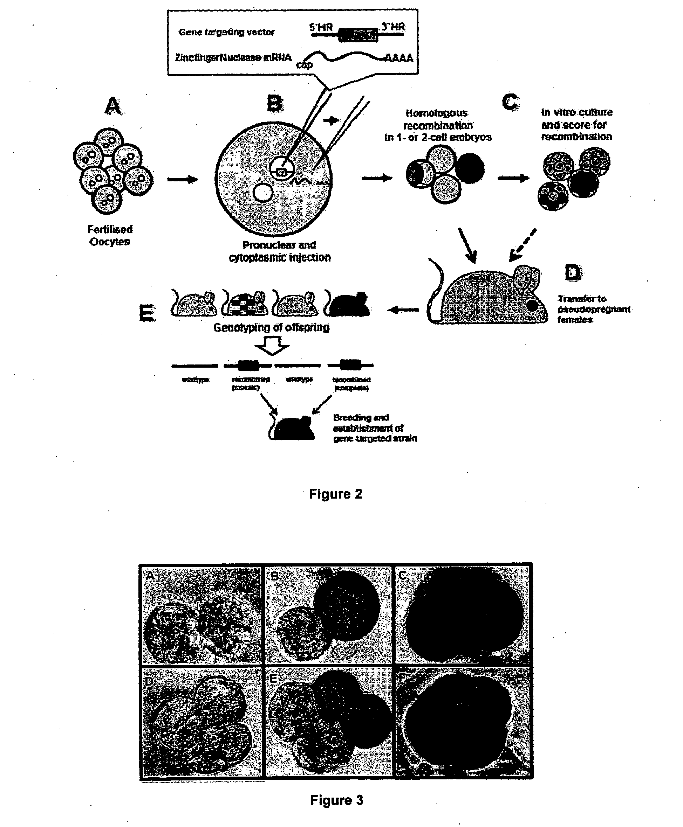

**Figure 2****.** Scheme for the generation of genetically modified mice at the Rosa26 locus by injection of the pRosa26.5-2 gene targeting vector together with mRNA coding for Rosa26 specific zincfinger nucleases. A: Fertilised oocytes, collected from superovulated females; B: Microinjection of a gene targeting vector and mRNA coding for zincfinger nucleases into one pronucleus and the cytoplasm of a fertilised oocyte; C: In vitro culture of injected embryos and assessment of reporter gene activity. Injected embryos can either directly transferred to pseudopregnant females or after detection of the reporter activity if a live stain is used; D: Pseudopregnant females deliver live offspring from microinjected oocytes, E: The offspring is genotyped for the presence of the induced genetic modification. Positive animals are selected for further breeding to establish a gene targeted strain.
**Figure 3****.** Pre-implantation mouse embryos, injected at the zygote stage with the pRosa26.8-2 gene targeting vector and mRNA coding for Rosa26 specific Zincfinger nucleases, that were cultured *in vitro,* fixed and incubated with X-Gal staining solution to identify β-Galactosidase positive cells as indication for the occurrence of homologous recombination. A: non-stained 2-cell embryo; B: mosaic stained 2-cell embryo; C: complete stained 2-cell embryo; D: non-stained 4-cell embryo; E: mosaic stained 4-cell embryo; F: complete stained 4-cell embryo

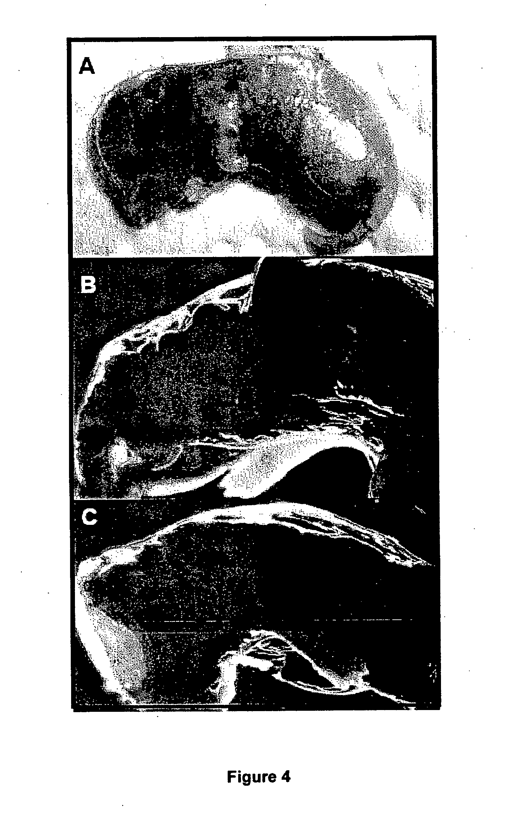

**Figure 4****.** Mouse fetus at day 18 of development, injected at the one cell (zygote) stage with the pRosa26.8-2 gene targeting vector and mRNA coding for Rosa26 specific Zincfinger nucleases.
   A: Macroscopic view before fixation and X-Gal staining; B and C: Embryo shown in A) upon fixation and X-Gal staining. Tissues exhibit a complete or mosaic pattern of β-Galactosidase activity.

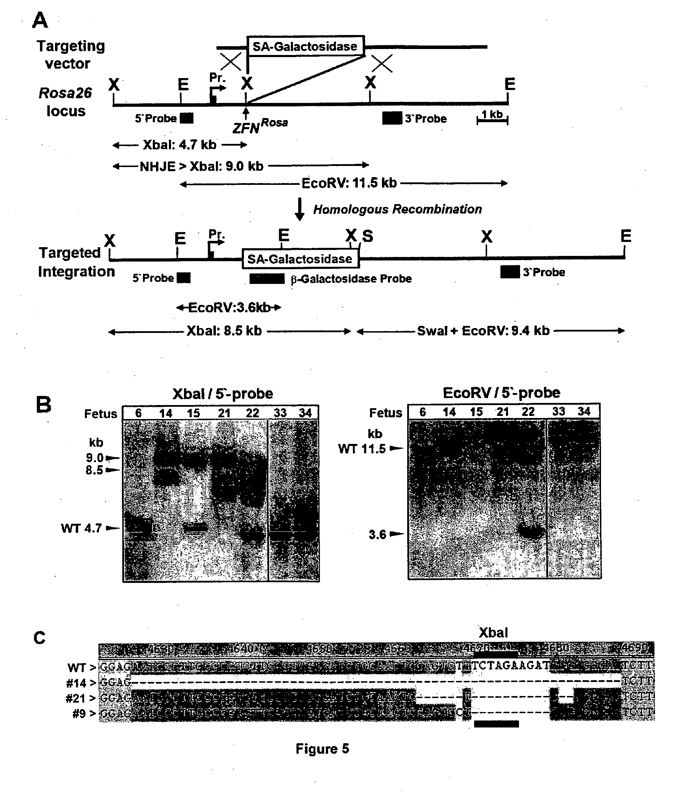

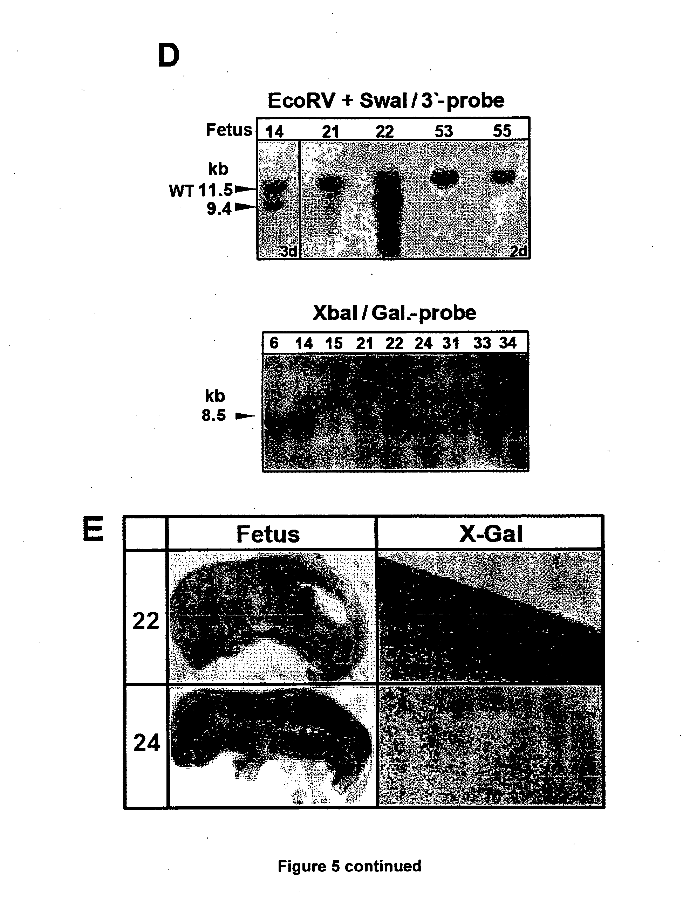

**Figure 5****.** Targeted integration of a β-Galactosidase reporter gene into the *Rosa26* locus.
   (A) Targeting vector pRosa26.8-2 for insertion of a 4.2 kb β-Galactosidase gene, including a splice acceptor (SA) and polyA site, into the *Rosa26* locus. The structure of the wildtype locus, including the ZFN^{Rosa} recognition sites that overlap with an intronic XbaI site (X), and of the recombined *Rosa26* allele are shown. The location of the *Rosa26* promoter (Pr.), first exon, of Southern blot probes and EcoRV (E), XbaI (X) and SwaI (S) sites and fragments are indicated. (B) Genomic DNA of E18 fetuses derived from zygote co-injections of ZFN^{Rosa} and pRosa26.8 was digested with XbaI or EcoRV and analyzed by Southern blotting using the *Rosa26* 5'-probe. The wildtype *Rosa26* locus exhibits a 4.7 kb Xbal and a 11.5 kb EcoRV fragment (WT). The loss of the ZFN^{Rosa} target Xbal site by NHEJ results in a 9 kb fragment (fetus 14, 15, 21). Targeted integration of the reporter gene is detected with the *Rosa26* 5'-probe by the presence of a 8.5 kb Xbal and 3.6 kb EcoRV fragment (fetus 22). (C) Sequence comparison of cloned PCR products (primers Rosa 5HA/Rosa 3HA), covering the ZFN^{Rosa} target region, from genomic DNA of fetus #9, #14 and #21, with the respective *Rosa26* wildtype sequence. The location of the XbaI site within the ZFN^{Rosa} target region is indicated. In PCR amplified *Rosa26* alleles from pups #9, #14 and #21 the Xbal site is lost by the deletion of multiple nucleotides (dashes). Sequence identity of compared sequences is marked; deleted or additional nucleotides, as compared to the *Rosa26* wildtype sequence, are indicated by a dash (-). (D) Southern blot analysis of EcoRV + Swal digested genomic DNA with the *Rosa26* 3'-probe reveals a 11.5 kb band derived from the wildtype locus and a 9.4 kb fragment from the recombined allele (fetus 22, 14). Exposure time for samples 21 to 55 were 2 days (2d) and 3 days (3d) for sample 14. Southern blot analysis of Xbal digested DNA of fetus 22 with an internal β-Galactosidase probe shows the predicted 8.5 kb band representing the recombined allele. **(E)** Habitus of fetus 22 (recombined) and 24 (wildtype) and X-Gal staining of liver sections.

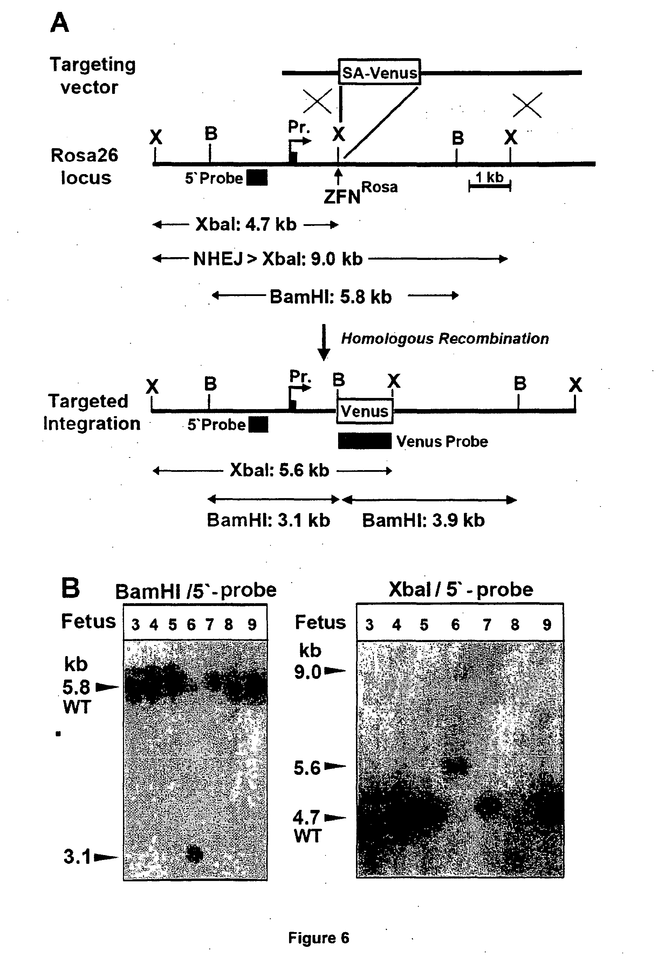

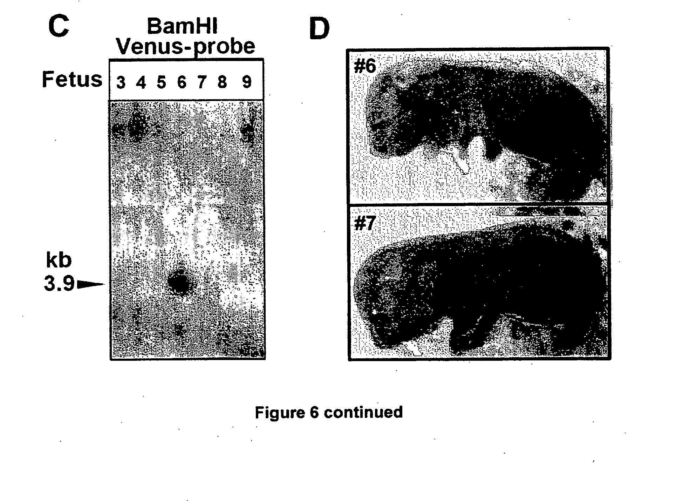

**Figure 6****.** Targeted integration of a Venus reporter gene into the *Rosa26* locus.
   **(A)** Targeting vector pRosa26.3-3 for insertion of a 1.1 kb Venus gene, including a splice acceptor (SA) and polyA site, into the *Rosa26* locus. The structure of the wildtype locus, including the ZFN^{Rosa} recognition sites that overlap with an intronic Xbal site (X), and of the recombined *Rosa26* allele are shown. The location of the *Rosa26* promoter (Pr.), first exon, of Southern blot probes and Xbal (X) and BamHI (B) sites and fragments are indicated. **(B)** Genomic DNA of E18 fetuses derived from zygotes coinjections of ZFN^{Rosa} and pRosa26.3 was digested with BamHI or Xbal and analyzed by Southern blotting using the *Rosa26* 5'-probe. The wildtype *Rosa26* locus exhibits a 5.8 kb BamHI and a 4.7 kb Xbal band. Targeted integration of the reporter gene is indicated by the presence of a predicted 3.1 kb BamHI and a 5.6 kb Xbal fragment detected with the *Rosa26* 5'-probe (fetus 6). The loss of the ZFN^{Rosa} target XbaI site by NHEJ in nonrecombined alleles results in a 9 kb band (fetus 6). **(C)** Analysis of BamHI digested DNA with the internal Venus probe shows a predicted 3.9 kb band from the recombined allele of fetus 6. **(D)** Habitus of fetus #6 (recombined) and #7 (wildtype).

The examples illustrate the invention.

### Example 1: Methods

### Plasmid constructions

The gene targeting vector pRosa26.8-2 (SEQ ID No:1) was derived from the vector pRosa26.8 by the removal of a 1.6 kb fragment that contains a pgk-diphtheria toxin A gene. For this purpose pRosa26.8 was digested with EcoRI and KpnI, the vector ends were blunted by treatment with Klenow and T4 DNA polymerase, and the 12.4 kb vector fragment was religated. pRosa26.8 was derived from pRosa26.1 (Soriano P.; Nat Genet 1999; 21:70-71) by insertion of a I-SceI recognition site into the SacII site located upstream of the 5' Rosa26 homology arm and the insertion of a splice acceptor element linked to the coding region for β-galactosidase and a polyadenylation signal downstream of the 5' homology arm. The expression vectors for zincfinger nucleases recognising a target site within the first intron of the murine Rosa26 locus (SEQ ID No: 2) were obtained from Sigma-Aldrich Inc..
Targeting vector pROSA26.3-3 (SEQ ID No: 3) is equal to pRosa26.8, except that it contains a 1.1 kb reporter cassette for expression of the Venus GFP protein. The homology arms of both targeting vectors were sequenced and found identical to the genomic sequence of the strain C57BL/6J.

### Preparation of DNA and RNA for microinjection

Plasmid pRosa26.8-2 was linearised by digestion with I-SceI, precipitated and resolved in injection buffer (10 mM Tris, 0.1 mM EDTA, pH 7.2). Targeting vector pRosa26.3-3 was used as circular DNA. Zincfinger nuclease RNA for injection was prepared from the linearised expression plasmids and transcribed from the T7 promoter using the mMessage mMachine kit (Ambion) according to the manufacturers instructions: The mRNA was further modified by the addition of a poly-A tail using the Poly(A) tailing kit and purified with MegaClear columns from Ambion. Finally the mRNA was precipitated and resolved in injection buffer. Aliquots for injection experiments were adjusted to a concentration of 5ng/µl of pRosa26.8-2 or 15 ng/ul of pRosa26.3-3 and 2.5 ng/µl of each zincfinger nuclease (ZFN) mRNA.

### Isolation and injection of fertilised oocytes

To isolate fertilised oocytes, males of the C57BL/6 strain were mated to super-ovulated females of the FVB strain. For super-ovulation three-week old FVB females were treated with 2.5 IU pregnant mares serum (PMS) 2 days before mating and with 2.5 IU Human chorionic gonadotropin (hCG) at the day of mating. Fertilised oocytes were isolated from the oviducts of plug positive females and microinjected in M2 medium (Sigma-Aldrich Inc Cat. No. M7167) with the pRosa26.8-2/ZFN mRNA preparation into one pronucleus and the cytoplasm following standard procedures (Nagy A, Gertsenstein M, Vintersten K, Behringer R., 2003. Manipulating the Mouse Embryo. Cold Spring Harbour, New York: Cold Spring Harbour Laboratory Press).

### In vitro culture and X-Gal staining of embryos

For the detection of β-galactosidase activity the microinjected oocytes were further cultivated for 3 days in KSOM medium (Millipore, Cat. No. MR-020-PD) at 37°C/5% CO₂/5% O₂ and fixed for 10 minutes in 4% formaldehyde in phosphate buffered saline (PBS). After washing with PBS the embryos were transferred to X-Gal staining solution (5 mM K3(Fe^{III}(CN)₆) 5 mM K4(Fe^{II}(CN)₆), 2 mM MgCl₂, 1 mg/ml X-Gal (5-bromo-chloro-3-indoyl-ß-D-galactopyranosid) in PBS) and incubated at 37°C for up to 24 hours.

### Southern blot analysis

6 µg of genomic DNA were digested overnight with 30 units restriction enzyme in a volume of 30 µl and then re-digested with 10 units enzyme for 2-3 hours. Samples were loaded on 0.8% agarose gels in TBE buffer and run at 55 V overnight. The gels were then denaturated for one hour in 1.5 M NaCl; 0.5 M NaOH, neutralized for one hour in 0.1 M Tris HCl pH 7.5; 0.5 M NaCl, washed with 2x SSC and blotted overnight with 20x SSC on Hybond N⁺ membranes (GE Healthcare). The membranes were then washed with 2x SSC, UV-crosslinked and stored at -20°C. For hybridization the membranes were pre-incubated in Church buffer (1% BSA, 1 mM EDTA, 0.5 M phosphate buffer, 7% SDS) for 1 hour at 65°C under rotation. The *Rosa26* 5'-probe (SEQ ID No: 4) was isolated as 460 bp EcoRI fragment from plasmid pCRII-Rosa5'-probe, the Rosa26 3'-probe (SEQ ID No: 5) was isolated as 660 bp EcoRI fragment from the plasmid pCRII-Rosa3'-probe, as described (Hitz *et al.* 2007). As β-Galactosidase probe (SEQ ID No: 6) we used the 1250 bp SacII/EcoRV coding fragment from pCMVβ and as Venus probe the venus coding region, isolated as 730 bp BamHI/EcoRI fragment (SEQ ID No: 7) from pCS2-venus, was used. DNA fragments used as hybridization probes were heat denatured and labeled with P³² marked dCTP (Perkin Elmer) using the high-prime DNA labeling kit (Roche). Labeled probe DNA was purified on MicroSpin™ S-200 HR columns (GE Healthcare), heat denatured, added to the hybridization buffer and membranes rotated overnight at 65°C. The washing buffer (2x SSC, 0,5% SDS) was pre-warmed to 65°C and the membranes were washed three times (five minutes, 30 minutes, 15 minutes) a 65°C under shaking. Next, the membranes were exposed at -80°C to Biomax MS1 films and enhancing screens (Kodak) for 1 to 5 days until development. Photos of autoradiographs were taken with a digital camera (Canon) on a transmitting light table and segments excised with the Adobe Photoshop software.

### PCR and sequence analysis

To analyze the Rosa26 alleles recombined with pRosa26.8-2 we amplified DNA from fetuses 22, 21 and 14 (co-injected with ZFN^{Rosa} and pRosa26.8) using the primer pairs Rosa 5HA (SEQ ID No: 8) and SA1 (SEQ ID No: 9) or SA2 (SEQ ID No: 10) to amplify the 5'-homology arm/splice acceptor junction and the primer pairs Rosa 3HA (SEQ ID No: 11) and pA1 (SEQ ID No: 12) or pA2 (SEQ ID No: 13) to amplify the junction of the SV40 polyA/3'-homology arm. Amplification was performed using Herculase polymerase (Invitrogen) in 100 µl reactions with 38 cycles of: 95°C -30s; 59°C -1 min; 72°C -1 min. In embryos with recombination events PCR products have the expected sizes: Rosa 5HA/SA1: 346 bp; Rosa 5HA/SA2: 365 bp; Rosa 3HA/pA1: 467 bp; Rosa 3HA/pA2: 625 bp. PCR products from embryos exhibiting homologous recombination events were directly sequenced (Sequiserve, Vaterstetten, Germany) and compared to the pRosa26.8 vector sequence using the Vector NTI software (Invitrogen).
Genomic DNA from fetuses 9, 14 and 21, that lost the Xbal site in the ZFN target region as found by Southern blotting, and of wildtype controls were further analysed for NHJE induced mutations by PCR amplification using the primer pair Rosa 5HA and Rosa 3HA (wildtype PCR product: 252 bp). These PCR products were cloned using the StrataClone Blunt PCR Cloning Kit (Stratagene). Plasmid DNA was isolated with the Qiaprep Spin Miniprep Kit (Qiagen) and analyzed by digestion with EcoRI to confirm the presence of vector inserts and digested with XbaI to analyze for the loss of the ZFN^{Rosa} target XbaI site. Selected clones were sequenced using the T3 forward primer (Sequiserve) and analyzed for mutations in comparison to the *Rosa26* wildtype sequence using the Vector NTI software.
To analyze Rosa26 homologous recombined alleles with pRosa26.3-3 we amplified DNA from fetus 6 (co-injected with ZFN^{Rosa} and pRosa26.3) using the primer pair Rosa 5HA and pA3 (SEQ ID No: 14) and the primer pair Rosa 3HA and SA3 (SEQ ID No: 15). PCR products (5HA/pA3: 1180 bp, 3HA/SA3: 1057 bp) were directly sequenced with a single 950 bp read (Sequiserve, Vaterstetten, Germany) and compared to the pRosa26.3-3 vector sequence using the Vector NTI software (Invitrogen).

### Preparation of genomic DNA

A segment of the other embryo half not used for staining was used for the isolation of genomic DNA. For this purpose frozen tissue segments were homogenized in lysis buffer (50 mM Tris-HCl, pH 8.0; 100 mM EDTA, pH 8,0; 1% SDS; 100 mM NaCl; 350 µg/µl Proteinase K) and incubated at 55°C for two hours under vigorous shaking. Upon tissue lysis phenol/chloroform (1:1) was added, the mixture was vortexed and centrifugated for ten minutes at 16.000 g. The supernatant was transferred into new tubes and extracted with chloroform. The supernatant was mixed with 0.7 volumes isopropanol to precipitate the genomic DNA. Precipitated DNA was collected by centrifugation and the pellet washed with 70% ethanol. The pellet was air-dried, re-suspended in TE buffer and incubated overnight at room temperature.

### Example 2: Targeted insertion of a β-galactosidase reporter gene into the Rosa26 locus.

With this experiment it was tested whether homologous recombination at the site of a double strand break induced by a zincfinger nuclease occurs at a reasonable frequency (>1%) in fertilised mouse oocytes. For this purpose the gene targeting vector pRosa26.8-2 that inserts via homology regions a reporter gene cassette was constructed, comprising a splice acceptor element, the coding region of β-galactosidase and a polyadenylation sequence, into the first intron of the *Rosa26* locus (Figure 1A, B). The *Rosa26* locus is a region on chromosome 6 that has been found to be ubiquitously expressed in all tissues and developmental stages of the mouse and is suitable for transgene expression (Zambrowicz BP, Imamoto A, Fiering S, Herzenberg LA, Kerr WG, Soriano P.; Proc Natl Acad Sci U S A 1997; 94:3789-3794; Seibler J, Zevnik B, Kuter-Luks B, Andreas S, Kern H, Hennek T, Rode A, Heimann C, Faust N, Kauselmann G, Schoor M, Jaenisch R, Rajewsky K, Kuhn R, Schwenk F.; Nucleic Acids Res 2003; 31:e12.). Upon recombination the vector splice acceptor is spliced to the donor site of the Rosa26 transcript such that the fusion transcript codes for β-galactosidase (Figure 1 C).

### A) Results

The linearised targeting vector was microinjected into fertilised mouse oocytes (Figure 2A, B) together with *in vitro* transcribed mRNA coding for a pair of zincfinger nuclease proteins that recognise the genomic sequence of Rosa26 and induce a double strand break at the insertion site of the reporter gene cassette (Figure 1B). Upon microinjection the zincfinger nuclease mRNAs are translated into proteins that induce a double strand break at one or both Rosa26 alleles in one or more cells of the developing embryo. This event stimulates the recombination of the pRosa28-2 vector with a Rosa26 allele via the homology regions present in the vector and leads to the site-specific insertion of the non-homologous reporter gene cassette into the genome (Figure 1C). Depending on the timing of these events recombination may occur within the one cell embryo or later in only a single cell of a 2-cell, 4-cell or 8-cell embryo. To detect such successful recombination events the microinjected zygotes were further cultivated *in vitro* and finally incubated with X-Gal as a β-galactosidase substrate that is converted into a insoluble blue coloured product (Figure 2C).

In both of two independent microinjection experiments we observed a high frequency of X-Gal stained embryos indicating the occurrence of homologous recombination at the one cell stage or at a later developmental stage. In the first experiment 10 of 47 (i.e. 21%) embryos showed partial or complete blue staining while in the second experiment 21 of 44 (i.e. 48%) embryos were positive. Representative non-stained, partially or completely positive embryos of the 2-and 4-cell stages are shown in Figure 3. Since these embryos were fixed before the staining procedure it was not possible to further derive mice from them.

### B) Generation of live mice carrying the reporter gene cassette

In further experiments, the microinjected zygotes were transferred into pseudopregnant females to allow their further development into live mice (Nagy A, Gertsenstein M, Vintersten K, Behringer R., 2003. Manipulating the Mouse Embryo. Cold Spring Harbour, New York: Cold Spring Harbour Laboratory Press). These experiments showed that the microinjected zygotes were able to develop into mouse embryos (Figure 4A) and that the integrated reporter gene is expressed. In one such experiment, 20 microinjected zygotes were transferred into a pseudopregnant female mouse and 6 embryos recovered at day 18 of development. The embryos were euthanized, cut into half and one half was stained with X-Gal staining solution as described above. This analysis revealed that one of six embryos was clearly and strongly positive for β-Galactosidase reporter gene activity, as indicated by the blue reaction product (Figure 4B,C).

### C) Analysing for successful genomic modification

In further experiments genomic DNA was extracted from embryonic and adult mice derived from microinjected zygotes. This DNA was then analysed for the expected homologous recombination event at the *Rosa26* locus by Southern blot analysis using a labelled probe located upstream of the 5' Rosa26 homology arm of the pRosa26.8-2 vector. The *Rosa26* wildtype allele can be recognised by a band of 11.5 kb while recombined mice can be identified by the presence of an additional band of 3.6 kb.
Southern blot analysis revealed the frequent loss of the Xbal site within the ZFN^{Rosa} target sequence in one or both Rosa26 alleles, as indicated by the presence of a predicted 9 kb fragment in addition to the 4.7 kb wildtype band (e.g. Fig. 5B). Among 58 pups derived from these injections we found 8 heterozygous and 5 homozygous XbaI negative genotypes (Fig. 5B), indicating that mutagenic NHEJ events were induced by ZFN^{Rosa} in 22% of one-cell embryos. One of these pups (#15, Fig. 5B) had an intermediate pattern showing a majority of XbaI negative and a minor proportion of wildtype alleles, indicating that the ZFNs action occurred in this embryo after the first cell division. Sequence analysis of PCR amplified target regions confirmed that the loss of the Xbal site is caused by the deletion of multiple nucleotides within the ZFN target region (Fig. 5C). The DNA of pup #22 (Fig. 5E) showing a 8.5 kb Xbal band (Fig. 5B), predicted for homologous recombination (Fig. 5A) was further characterized by Southern blotting. This analysis proved a bona fide homologous recombination event, as indicated by the presence of the predicted 3.6 kb EcoRV and 9.4 kb EcoRV/SwaI fragments for pup #22 (Fig. 5B, D), detected with external 5'- and 3'-hybridization probes, respectively. The complete integration of the reporter gene in pup #22 was confirmed with an internal β-Galactosidase-specific probe that detected the predicted 8.5 kb Xbal band (Fig. 5D). The analysis of liver sections for β-Galactosidase activity proved the functionality and heterozygous state of the targeted allele in pup#22 (Fig. 5E). In addition, we verified the integrity of the recombined locus by sequence analysis of the Rosa26/reporter junction regions amplified by PCR from pup #22. Among the 58 analysed pups we found two, # 14 and 21, that exhibit unexpected Xbal fragments of 4.7- 8.5 kb (Fig. 5B), were further shown by Southern blot (Fig. 5D, #14) and PCR analysis to contain 3'-sequences, but not the coding region of the reporter cassette (Fig. 5D) and represent incomplete or rearranged recombination products.

### D) Targeted Integration of a Venus reporter gene into the Rosa26 locus

In a second experiment we used the targeting vector pRosa26.3-3 that inserts a 1.1 kb *Venus* reporter gene into the *Rosa26* locus (Fig. 6A). DNA analysis of 22 pups derived from zygote co-injections of this vector with ZFN^{Rosa} mRNAs revealed the presence of one recombined allele, as shown by a 3.1 kb BamHI and a 5.6 kb XbaI fragment (Fig. 6B, fetus 6, Fig. 6D) detected with the 5'-Rosa probe and by a 3.9 kb BamHI band detected with a Venus probe (Fig 6C). The Venus reporter cassette was amplified from the genomic DNA of pup #6 and was found by sequence analysis entirely intact. The second Rosa26 allele of pup #6 had lost the XbaI site within the ZFN^{Rosa} target sequence, as indicated by the presence of a 9 kb Xbal fragment and the absence of the wildtype 4.7 kb band (Fig. 6B), detected with the *Rosa26* 5'-probe.

### References

Bloch, K.D. (2001). Mapping by multiple endonuclease digestions. Curr Protoc Mol Biol Chapter 3, Unit 3.2.
Bradley A, Evans M, Kaufman MH, Robertson E. 1984. Formation of germ-line chimaeras from embryo-derived teratocarcinoma cell lines. Nature 309:255-256.
Brinster RL, Braun RE, Lo D, Avarbock MR, Oram F, Palmiter RD. 1989. Targeted correction of a major histocompatibility class II E alpha gene by DNA microinjected into mouse eggs. Proc Natl Acad Sci U S A 86:7087-7091.
Capecchi MR. 1989. The new mouse genetics: altering the genome by gene targeting. Trends Genet 5:70-76.
Capecchi MR. 2005. Gene targeting in mice: functional analysis of the mammalian genome for the twenty-first century. Nat Rev Genet 6:507-512.
Cheah SS, Behringer RR. 2000. Gene-targeting strategies. Methods Mol Biol 136:455-463.
Collins FS, Rossant J, Wurst W. 2007. A mouse for all reasons. Cell 128:9-13.
DeChiara TM. 2001. Gene targeting in ES cells. Methods Mol Biol 158:19-45.
Doyon Y, McCammon JM, Miller JC, Faraji F, Ngo C, Katibah GE, Amora R, Hocking TD, Zhang L, Rebar EJ, Gregory PD, Urnov FD, Amacher SL. 2008. Heritable targeted gene disruption in zebrafish using designed zinc-finger nucleases. Nat Biotechnol 26:702-708.
Durai S, Mani M, Kandavelou K, Wu J, Porteus MH, Chandrasegaran S. 2005. Zinc finger nucleases: custom-designed molecular scissors for genome engineering of plant and mammalian cells. Nucleic Acids Res 33:5978-5990.
Evans MJ, Kaufman MH. 1981. Establishment in culture of pluripotential cells from mouse embryos. Nature 292:154-156.
Geurts AM, Cost GJ, Freyvert Y, Zeitler B, Miller JC, Choi VM, Jenkins SS, Wood A, Cui X, Meng X, Vincent A, Lam S, Michalkiewicz M, Schilling R, Foeckler J, Kalloway S, Weiler H, Menoret S, Anegon I, Davis GD, Zhang L, Rebar EJ, Gregory PD, Urnov FD, Jacob HJ, Buelow R. 2009. Knockout rats via embryo microinjection of zinc-finger nucleases. Science 325:433.
Gong M, Rong YS. 2003. Targeting multi-cellular organisms. Curr Opin Genet Dev 13:215-220.
Gu H, Marth JD, Orban PC, Mossmann H, Rajewsky K. 1994. Deletion of a DNA polymerase beta gene segment in T cells using cell type-specific gene targeting. Science 265:103-106.
Hasty P, Abuin A, Bradley A. 2000. Gene targeting, principles, and practice in mammalian cells. In: Joyner AL, editor. Gene Targeting: a practical approach, 2nd ed. Oxford: Oxford University Press. p 1-35.
Hockemeyer D, Soldner F, Beard C, Gao Q, Mitalipova M, DeKelver RC, Katibah GE, Amora R, Boydston EA, Zeitler B, Meng X, Miller JC, Zhang L, Rebar EJ, Gregory PD, Urnov FD, Jaenisch R. 2009. Efficient targeting of expressed and silent genes in human ESCs and iPSCs using zinc-finger nucleases. Nat Biotechnol 27:851-857.
Hitz, C. Wurst, W. and Kühn, R. 2007. Conditional brain-specific knockdown of MAPK using Cre/loxP regulated RNA interference. Nucleic Acids Res. 35, e90.
Ivarie R. 2006. Competitive bioreactor hens on the horizon. Trends Biotechnol 24:99-101.
Kamihira M, Nishijima K, lijima S. 2004. Transgenic birds for the production of recombinant proteins. Adv Biochem Eng Biotechnol 91:171-189.
Lai L, Prather RS. 2003. Creating genetically modified pigs by using nuclear transfer. Reprod Biol Endocrinol 1:82.
Nagy A, Gertsenstein M, Vintersten K, Behringer R. 2003. Manipulating the Mouse Embryo, third edition ed. Cold Spring Harbour, New York: Cold Spring Harbour Laboratory Press.
Nothias JY, Majumder S, Kaneko KJ, DePamphilis ML. 1995. Regulation of gene expression at the beginning of mammalian development. J Biol Chem 270:22077-22080.
Nothias JY, Miranda M, DePamphilis ML. 1996. Uncoupling of transcription and translation during zygotic gene activation in the mouse. EMBO J 15:5715-5725.
Palmiter RD, Brinster RL. 1986. Germ-line transformation of mice. Annu Rev Genet 20:465-499.
Paques F, Haber JE. 1999. Multiple pathways of recombination induced by double-strand breaks in Saccharomyces cerevisiae. Microbiol Mol Biol Rev 63:349-404.
Peippo, J., Viitala, S., Virta, J., Raty, M., Tammiranta, N., Lamminen; T., Aro, J., Myllymaki, H., and Vilkki, J. (2007). Birth of correctly genotyped calves after multiplex marker detection from bovine embryo microblade biopsies. Mol Reprod Dev 74, 1373-1378.
Porteus MH, Baltimore D. 2003. Chimeric nucleases stimulate gene targeting in human cells. Science 300:763.
Porteus MH, Carroll D. 2005. Gene targeting using zinc finger nucleases. Nat Biotechnol 23:967-973.
Rouet P, Smih F, Jasin M. 1994. Expression of a site-specific endonuclease stimulates homologous recombination in mammalian cells. Proc Natl Acad Sci U S A 91:6064-6068.
Rouet P, Smih F, Jasin M. 1994. Introduction of double-strand breaks into the genome of mouse cells by expression of a rare-cutting endonuclease. Mol Cell Biol 14:8096-8106.
Santiago Y, Chan E, Liu PQ, Orlando S, Zhang L, Urnov FD, Holmes MC, Guschin D, Waite A, Miller JC, Rebar EJ, Gregory PD, Klug A, Collingwood TN. 2008. Targeted gene knockout in mammalian cells by using engineered zinc-finger nucleases. Proc Natl Acad Sci U S A 105:5809-5814.
Schwartzberg PL, Goff SP, Robertson EJ. 1989. Germ-line transmission of a c-abl mutation produced by targeted gene disruption in ES cells. Science 246:799-803.
Seibler J, Zevnik B, Kuter-Luks B, Andreas S, Kern H, Hennek T, Rode A, Heimann C, Faust N, Kauselmann G, Schoor M, Jaenisch R, Rajewsky K, Kuhn R, Schwenk F. 2003. Rapid generation of inducible mouse mutants. Nucleic Acids Res 31:e12.
Soriano P. 1999. Generalized lacZ expression with the ROSA26 Cre reporter strain. Nat Genet 21:70-71.
te Riele H, Maandag ER, Bems A. 1992. Highly efficient gene targeting in embryonic stem cells through homologous recombination with isogenic DNA constructs. Proc Natl Acad Sci U S A 89:5128-5132.
Thomas KR, Capecchi MR. 1987. Site-directed mutagenesis by gene targeting in mouse embryo-derived stem cells. Cell 51:503-512.
Torres RM, Kühn R. 1997. Laboratory protocols for conditional gene targeting. Oxford: Oxford University Press. 167 p.
Urnov FD, Miller JC, Lee YL, Beausejour CM, Rock JM, Augustus S, Jamieson AC, Porteus MH, Gregory PD, Holmes MC. 2005. Highly efficient endogenous human gene correction using designed zinc-finger nucleases. Nature 435:646-651.
Zambrowicz BP, Imamoto A, Fiering S, Herzenberg LA, Kerr WG, Soriano P. 1997. Disruption of overlapping transcripts in the ROSA beta geo 26 gene trap strain leads to widespread expression of beta-galactosidase in mouse embryos and hematopoietic cells. Proc Natl Acad Sci U S A 94:3789-3794.

## Claims

1. A method of modifying a target sequence in the genome of a non-human mammalian or avian oocyte by homologous recombination with a donor nucleic acid sequence, the method comprising the steps:
(a) introducing into the oocyte a zinc finger nuclease or a nucleic acid molecule encoding the zinc finger nuclease in expressible form, wherein the zinc finger nuclease specifically binds within the target sequence and introduces a double strand break within the target sequence; and
(b) introducing a nucleic acid molecule into the oocyte, wherein the nucleic acid molecule comprises the donor nucleic acid sequence and regions homologous to the target sequence.

2. The method of claim 1, wherein the non-human mammalian or avian oocyte is a fertilised mammalian or avian oocyte.

3. The method of claim 1 or 2, wherein the zinc finger nuclease and the nucleic acid molecule are introduced into the oocyte by microinjection.

4. The method of any one of claims 1 to 3, wherein the nucleic acid molecule encoding the zinc finger nuclease in expressible form is mRNA.

5. The method of any one of claims 1 to 4, wherein the regions homologous to the target sequence are localised at the 5' and 3' end of the donor nucleic acid sequence.

6. The method of any one of claims 1 to 5, wherein the regions homologous to the target sequence comprised in the nucleic acid molecule have a length of at least 400 bp.

7. The method of any one of claims 1 to 6, wherein the modification of the target sequence is selected from the group consisting of substitution, insertion and deletion of a least one nucleotide of the target sequence.

8. The method of any one of claims 1 to 7, wherein the non-human oocyte is from a mammal selected from the group consisting of rodents, dogs, felides, primates, rabbits, pigs, or cows or wherein the oocyte is from an avian selected from the group consisting of chickens, turkeys, pheasants, ducks, geese, quails and ratites including ostriches, emus and cassowaries.

9. A method of producing a non-human mammal or an avian carrying a modified target sequence in its genome, the method comprising:
(a) modifying the target sequence in the genome of a mammalian or avian oocyte in accordance with any one of claims 1 to 8; and
(b) analysing the offspring delivered by the non-human female host to which the oocyte obtained in (a) has been transferred for the presence of the modification.

10. The method of claim 9, wherein the non-human mammal is selected from the group consisting of rodents, dogs, felides, primates, rabbits, pigs and cows or wherein the avian is selected from the group consisting of chickens, turkeys, pheasants, ducks, geese, quails and ratites including ostriches, emus and cassowaries.

## Patentansprüche

1. Verfahren zum Verändern einer Zielsequenz im Genom einer nicht-menschlichen Säuger- oder Vogel-Oozyte durch homologe Rekombination mit einer Donor-Nukleinsäuresequenz, wobei das Verfahren die Schritte umfasst:
(a) Einbringen einer Zink-Finger-Nuklease, oder eines Nukleinsäuremoleküls, das die Zink-Finger-Nuklease in exprimierbarer Form kodiert, in die Oozyte, wobei die Zink-Finger-Nuklease spezifisch in der Zielsequenz bindet und in der Zielsequenz einen Doppelstrangbruch einführt; und
(b) Einbringen eines Nukleinsäuremoleküls in die Oozyte, wobei das Nukleinsäuremolekül die Donor-Nukleinsäuresequenz und Bereiche umfasst, die homolog zur Zielsequenz sind.

2. Verfahren nach Anspruch 1, wobei die nicht-menschliche Säuger- oder Vogel-Oozyte eine befruchtete Säuger- oder Vogel-Oozyte ist.

3. Verfahren nach Anspruch 1 oder 2, wobei die Zink-Finger-Nuklease und das Nukleinsäuremolekül mittels Mikroinjektion in die Oozyte eingebracht werden.

4. Verfahren nach einem der Ansprüche 1 bis 3, wobei das Nukleinsäuremolekül, das die Zink-Finger-Nuklease in exprimierbarer Form kodiert, mRNA ist.

5. Verfahren nach einem der Ansprüche 1 bis 4, wobei die Bereiche, die homolog zur Zielsequenz sind, sich an den 5'- und 3'-Enden der Donor-Nukleinsäuresequenz befinden.

6. Verfahren nach einem der Ansprüche 1 bis 5, wobei die in dem Nukleinsäuremolekül umfassten Bereiche, die homolog zur Zielsequenz sind, eine Länge von mindestens 400 bp haben.

7. Verfahren nach einem der Ansprüche 1 bis 6, wobei die Veränderung der Zielsequenz der Austausch, das Einfügen oder das Entfernen von mindestens einem Nukleotid der Zielsequenz ist.

8. Verfahren nach einem der Ansprüche 1 bis 7, wobei die nicht-menschliche Säuger-Oozyte von einem Säuger ausgewählt aus der Gruppe bestehend aus Nagern, Hunden, Katzen, Primaten, Kaninchen, Schweinen oder Kühen ist oder wobei die Vogel-Oozyte von einem Vogel ausgewählt aus der Gruppe bestehend aus Hühnern, Truthähnen, Fasanen, Enten, Gänsen, Wachteln und Laufvögeln, einschließlich Straußen, Emus und Kasuaren ist.

9. Verfahren zum Herstellen eines nicht-menschlichen Säugers oder Vogels, der eine veränderte Zielsequenz in seinem Genom trägt, wobei das Verfahren umfasst:
(a) Verändern der Zielsequenz im Genom einer Säuger- oder Vogel-Oozyte gemäß einem der Ansprüche 1 bis 8; und
(b) Untersuchen der Nachkommen, die von dem nicht-menschlichen, weiblichen Wirt geboren werden, in den die in (a) erhaltene Oozyte eingebracht wurde, auf das Vorhandensein der Veränderung.

10. Verfahren nach Anspruch 9, wobei der nicht-menschliche Säuger ausgewählt ist aus der Gruppe bestehend aus Nagern, Hunden, Katzen, Primaten, Kaninchen, Schweinen und Kühen oder wobei der Vogel ausgewählt ist aus der Gruppe bestehend aus Hühnern, Truthähnen, Fasanen, Enten, Gänsen, Wachteln und Laufvögeln, einschließlich Straußen, Emus und Kasuaren.

## Revendications

1. Méthode permettant de modifier une séquence cible dans le génome d'un ovocyte de mammifère non humain ou d'oiseau par recombinaison homologue avec une séquence d'acide nucléique donneur, la méthode comprenant les étapes :
(a) d'introduction dans l'ovocyte d'une nucléase à doigt de zinc ou d'une molécule d'acide nucléique codant pour la nucléase à doigt de zinc sous une forme pouvant s'exprimer, dans laquelle la nucléase à doigt de zinc se lie spécifiquement à l'intérieur de la séquence cible et introduit une cassure double brin à l'intérieur de la séquence cible ; et
(b) d'introduction d'une molécule d'acide nucléique dans l'ovocyte, dans laquelle la molécule d'acide nucléique comprend la séquence d'acide nucléique donneur et des régions homologues à la séquence cible.

2. Méthode selon la revendication 1, dans laquelle l'ovocyte de mammifère non humain ou d'oiseau est un ovocyte fertilisé de mammifère ou d'oiseau.

3. Méthode selon la revendication 1 ou 2, dans laquelle la nucléase à doigt de zinc et la molécule d'acide nucléique sont introduites dans l'ovocyte par micro-injection.

4. Méthode selon l'une quelconque des revendications 1 à 3, dans laquelle la molécule d'acide nucléique codant pour la nucléase à doigt de zinc sous une forme pouvant s'exprimer est de l'ARNm.

5. Méthode selon l'une quelconque des revendications 1 à 4, dans laquelle les régions homologues à la séquence cible sont localisées aux extrémités 5' et 3' de la séquence d'acide nucléique donneur.

6. Méthode selon l'une quelconque des revendications 1 à 5, dans laquelle les régions homologues à la séquence cible comprises dans la molécule d'acide nucléique ont une longueur d'au moins 400 pb.

7. Méthode selon l'une quelconque des revendications 1 à 6, dans laquelle la modification de la séquence cible est choisie dans le groupe constitué d'une substitution, insertion et délétion d'au moins un nucléotide de la séquence cible.

8. Méthode selon l'une quelconque des revendications 1 à 7, dans laquelle l'ovocyte non humain provient d'un mammifère choisi dans le groupe constitué des rongeurs, chiens, félidés, primates, lapins, porcs ou vaches ou dans laquelle l'ovocyte provient d'un oiseau choisi dans le groupe constitué des poules, dindes, faisans, canards, oies, cailles et ratites y compris les autruches, émeus et casoars.

9. Méthode de production d'un mammifère non humain ou d'un oiseau portant une séquence cible modifiée dans son génome, la méthode comprenant :
(a) la modification de la séquence cible dans le génome d'un ovocyte de mammifère ou d'oiseau conformément à l'une quelconque des revendications 1 à 8 ; et
(b) l'analyse de la descendance mise au monde par l'hôte femelle non humain à qui l'ovocyte obtenu en (a) a été transféré pour la présence de la modification.

10. Méthode selon la revendication 9, dans laquelle le mammifère non humain est choisi dans le groupe constitué des rongeurs, chiens, félidés, primates, lapins, porcs ou vaches ou dans laquelle l'oiseau est choisi dans le groupe constitué des poules, dindes, faisans, canards, oies, cailles et ratites y compris les autruches, émeus et casoars.
